# EUROPEAN PATENT SPECIFICATION

(11) **EP 2 501 721 B1**
(45) Date of publication and mention of the grant of the patent: **15.11.2017**
(21) Application number: 10796094.0
(22) Date of filing: 17.11.2010
(51) Int. Cl.: C07K 16/00

(54) **MULTIVALENT ANTIBODIES COMPRISING FAB-DSFV**
POLYVALENTE ANTIKÖRPER MIT FAB-DSFV
ANTICORPS MULTIVALENTS COMPRENANT FAB-DSFV

(30) Priority: 17.11.2009 GB 0920127
(43) Date of publication of application: 26.09.2012
(73) Proprietor: UCB Biopharma SPRL, 1070 Brussels (BE)
(72) Inventor: HUMPHREYS, David Paul, Slough Berkshire SL1 3WE (GB); HEYWOOD, Sam Philip, Slough Berkshire SL1 3WE (GB); LAWSON, Alastair David Griffiths, Slough Berkshire SL1 3WE (GB)
(74) Representative: UCB Intellectual Property
(86) International application number: PCT/GB2010/002120
(87) International publication number: WO 2011/061492

(56) References cited:
- WO-A1-2008/038024
- WO-A1-2009/018386
- WO-A1-2009/040562
- WO-A1-2010/035012
- WO-A2-98/37200
- WO-A2-2004/081026
- WO-A2-2006/034488
- CHAPMAN ANDREW P: "PEGylated antibodies and antibody fragments for improved therapy: A review", ADVANCED DRUG DELIVERY REVIEWS, ELSEVIER BV, AMSTERDAM, NL, vol. 54, no. 4, 17 June 2002 (2002-06-17), pages 531-545, XP002300924, ISSN: 0169-409X, DOI: DOI:10.1016/S0169-409X(02)00026-1
- NATARAJAN A ET AL: "CHARACTERIZATION OF SITE-SPECIFIC SCFV PEGYLATION FOR TUMOR-TARGETING PHARMACEUTICALS", BIOCONJUGATE CHEMISTRY, ACS, WASHINGTON, DC, US, vol. 16, no. 1, 1 January 2005 (2005-01-01), pages 113-121, XP008048580, ISSN: 1043-1802, DOI: DOI:10.1021/BC0498121
- MELMED GIL Y ET AL: "Certolizumab pegol", NATURE REVIEWS. DRUG DISCOVERY, NATURE PUBLISHING GROUP, GB, vol. 7, no. 8, 1 August 2008 (2008-08-01), pages 641-642, XP009147828, ISSN: 1474-1784
- BLICK STEPHANIE K A ET AL: "Certolizumab pegol: in Crohn's disease.", BIODRUGS : CLINICAL IMMUNOTHERAPEUTICS, BIOPHARMACEUTICALS AND GENE THERAPY 2007 LNKD- PUBMED:17516714, vol. 21, no. 3, 2007, pages 195-201 ; DIS, XP009147825, ISSN: 1173-8804
- TON N C ET AL: "Phase I evaluation of CDP791, a PEGylated di-Fab' conjugate that binds vascular endothelial growth factor receptor 2.", CLINICAL CANCER RESEARCH : AN OFFICIAL JOURNAL OF THE AMERICAN ASSOCIATION FOR CANCER RESEARCH 1 DEC 2007 LNKD- PUBMED:18056191, vol. 13, no. 23, 1 December 2007 (2007-12-01), pages 7113-7118, XP009147881, ISSN: 1078-0432
- ZHU ET AL: "REMODELLING DOMAIN INTERFACES TO ENHANCE HETERODIMER FORMATION", PROTEIN SCIENCE, WILEY, US, vol. 6, no. 4, 1 April 1997 (1997-04-01), pages 781-788, XP002084764, ISSN: 0961-8368
- REITER Y ET AL: "STABILIZATION OF THE FV FRAGMENTS IN RECOMBINANT IMMUNOTOXINS BY DISULFIDE BONDS ENGINEERED INTO CONSERVED FRAMEWORK REGIONS", BIOCHEMISTRY, AMERICAN CHEMICAL SOCIETY, US, vol. 33, no. 18, 10 May 1994 (1994-05-10), pages 5451-5459, XP002008021, ISSN: 0006-2960, DOI: 10.1021/BI00184A014
- REITER Y ET AL: "ENGINEERING INTERCHAIN DISULFIDE BONDS INTO CONSERVED FRAMEWORK REGIONS OF FV FRAGMENTS: IMPROVED BIOCHEMICAL CHARACTERISTICS OF RECOMBINANT IMMUNOTOXINS CONTAINING DISULFIDE-STABILIZED FV", PROTEIN ENGINEERING, OXFORD UNIVERSITY PRESS, SURREY, GB, vol. 7, no. 5, 1 May 1994 (1994-05-01), pages 697-704, XP000443154, ISSN: 0269-2139

## Description

The present disclosure relates to antibodies with two antigen binding sites, for example wherein the steric hindrance around each site is minimized, such that affinity to the target antigen or antigens is not detrimentally affected by the format.

Multivalent antibodies are known. However, even though the basic concept was disclosed a number of years ago, there have been practical difficulties associated with exploiting the technology and thus it has not been widely adopted for the preparation of pharmaceutical biologic products in development.

WO2009/018386 discloses certain multispecific epitope binding proteins and methods of making and using the same. WO2009/040562 discloses a bispecific antibody format, referred to as a "FabFv".

A non-natural/non-native antibody format can be difficult to express, which may significantly increase the cost of goods to an untenable level. The formats may increase the immunogenicity or reduce the *in vivo* stability in comparison to a standard antibody or fragment and/or may have undesirable pharmacokinetics.

In particular the problems associated with preparing homogenous products have been a concern for non-natural formats. If, for example, there is more than one permutation for combining the component monomers then mixtures can result. Thus elaborate purification methods may be required to isolate the desired/target entity at satisfactory purity levels.

This has been addressed in a number of ways, for example using short linkers in the production of bispecific diabodies was said to aid appropriate dimerisation. However, data has shown that the orientation of the variable domains can influence expression of the format and the formation of active binding sites.

One approach to force the assembly in the desired arrangement or orientation is referred to as the "knob-in-hole" method, in which a large "knob" is introduced in the VH domain by, for example in some antibodies exchanging valine 137 with the large residue phenyl alanine and replacing leucine 45 with tryptophan. A complementary hole can be introduced, for example in the VL domain by, in some antibodies, mutating phenylalanine 98 to methionine and tryptophan 87 to alanine. However, reduced antigen-binding activity was observed for several constructs.

WO2010/035012 published April 2010 discloses a so-called disulfide stabilised "FabFv" molecule.In the present invention the provision of CL in the light chain and C_{H}1 in the heavy chain ensures the correct orientation of the chains. Thus there is provided a recombinant fusion protein comprising:
a heavy chain comprising, in sequence from the N-terminal, a variable domain nominally V_{H}1, a C_{H}1 region and a further variable domain nominally V_{H}2,
a light chain comprising, in sequence from the N-terminal, a variable domain nominally V_{L}1, a CL domain and a variable domain nominally V_{L}2,
wherein said heavy and light chains are aligned to provide a first binding site formed by a first variable domain pair of V_{H}1 and V_{L}1 and a second binding site formed by a second variable domain pair of V_{H}2 and V_{L}2,
wherein there is a disulfide bond between a variable domain pair of V_{H}2 and V_{L}2 between two cysteine residues selected from the group consisting of VH37 and VL95, VH44 and VL100, VH44 and VL105, VH45 and VL87, VH55 and VL101, VH100 and VL50, VH100b and VL49, VH98 and VL46, and, VH105 and VL43, such as wherein the cysteine of V_{H}2 is at position 44 and the cysteine of V_{L}2 is at position 100, and
said fusion protein is conjugated to one or two PEG polymer molecules through a solvent exposed cysteine, wherein the numbering is Kabat numbering of variable domains.

The recombinant fusion protein provided herein also advantageously has a half-life similar to that of a complete antibody and therefore is likely to be suitable for use in treatment.

### Brief Description of the Figures

Figure 1 shows various fusion protein formats according to the present disclosure.
Figure 2A shows various known antibody fragments formats
Figure 2B shows one possible arrangement for a dimer format according to the presently claimed invention
Figure 3 shows the light chain amino acid sequence for antibody 4D5
Figure 4 shows the heavy chain amino acid sequence for antibody 4D5.
Figure 5 shows A26Fab-(3xG4S)-dsFv645 with positions of the surface Cys mutations shown in bold
Figure 6 shows a non-reducing gel of PEGylated mutants of A26Fab-(3xG4S)-dsFv645, S182 and S163.

The heavy chain as employed herein is the chain which comprises a C_{H}1 domain.

In one embodiment the heavy chain only comprises one C_{H}1 domain.

V_{H}1 and V_{H}2 as employed herein is intended to refer to the fact that the variable regions are located in the heavy chain of the antibody according to the present invention. It is not in itself indicative of the origin of the variable region as such.

V_{L}1 and V_{L}2 as employed herein is intended to refer to the fact that the variable regions are located in the light chain of the antibody according to the present invention. It is not in itself indicative of the origin of the variable region as such.

The light chain as employed herein comprises a CL domain. In one embodiment the light chain only comprises one CL domain.

The arrangement herein of CL as the constant region fragment in the light chain and C_{H}1 as the constant region fragment in the heavy chain is thought to minimize inappropriate dimerisation.

In one embodiment the fusion protein according to the disclosure comprises a hinge, for example, as a linker to a variable domain.

The hinge may be attached to the C-terminal of C_{H}1 in a corresponding position to that found in full length antibody and may form a link between C_{H}1 and V_{H}2. In this embodiment a linker will also be provided on the light chain so V_{L}2 is appropriately placed to pair with V_{H}2 in the heavy chain. The linker employed in the light chain may be identical, similar or completely different to the hinge linker employed in the heavy chain.

In an alternative embodiment the light chain comprises a hinge, for example attached to the C-terminal of the CL domain and linking the CL domain with V_{L}2. In this embodiment a linker may be required in the heavy chain to ensure the V_{L}2 and V_{H}2 domains can pair appropriately to form an active site. The linker in the heavy chain can be identical, similar or completely different to the linker employed in the light chain.

Examples of suitable hinges are given below.

The variable domains are provided in each chain such that they form predefined pairs with suitable/adequate binding to a target antigen.

In one embodiment the variable domain pair has affinity for a target antigen of 100nm or less, such as 50nm or less, in particular 1 nm or less.

Suitable variable domains pairs may be identified by any means possible, for example including generation of antibodies in hosts with subsequent screening of B cells. Alternatively suitable pairs may be identified by phage display.

Phage display methods known in the art and include those disclosed by Brinkman et al., J. Immunol. Methods, 1995, 182, 41-50; Ames et al., J. Immunol. Methods, 1995, 184, 177-186; Kettleborough et al. Eur. J. Immunol., 1994, 24, 952-958; Persic et al., Gene, 1997 187, 9-18; and Burton et al., Advances in Immunology, 1994, 57, 191-280; WO 90/02809; WO 91/10737; WO 92/01047; WO 92/18619; WO 93/11236; WO 95/15982; and WO 95/20401; and US 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743; and 5,969,108.

Transgenic mice, or other organisms, including other mammals, may be used to generate humanized antibodies.

In one embodiment a variable domain pair (or each variable domain pair) is a cognate pair.

Cognate pair as employed herein is intended to refer to a natural pair of variable domains, that is to say isolated from a single antibody or antibody expressing cell.

In one example the cognate pair are a complementary VH/VL pair which bind the antigen co-operatively i.e. they are a complementary VH/VL pair. Typically the cognate pair will be a VH/VL pair derived from the same antibody.

In one example the cognate pair are a pair of variable domains isolated as a pair from a 'library of pairs', such as a Fab phage display library.

In one example the VH/VL pair are monospecific.

First and second binding sites are relative terms (relative to each other) and are nominal labels given to the binding sites to differentiate one from the other. If one binding site is labeled "the first" then the other is labeled "the second".

First cognate pair and second pair are also relative labels to nominally differentiate the pairs. One pair labeled "first pair" herein is not definitive for the position in the molecule.

Variable domains may have been optimized and/or humanized. Optimised/humanized variable domains derived from a cognate pair will still be considered a cognate pair after optimization/humanization.

CL as employed herein refers to the constant region portion in the light chain, which may be a naturally occurring light chain constant region.

Each variable domain may directly joined or joined via a linker to the constant domain in the relevant chain.

"Directly linked to" as employed herein is intended to refer to a continuous amino acid sequence that is uninterrupted, i.e. linked directly via a peptide bond, for example directly to the sequence of the variable domain or conversely the constant region fragment and not joined via a linker. Inserting a "non-natural peptide linker" into an amino acid sequence disrupts the sequence and thus a sequence containing a "non-natural peptide linker" is not considered to be fused to the relevant portions directly, within the meaning of the present specification. The addition a natural peptide linker would also be considered interruption of the amino acid sequence, if it cannot be considered to form part of the sequence of one or more of the relevant components, such as a variable domain or constant region fragment (such as the CL domain or C_{H}1 domain).

V_{H}1 will generally be joined directly to C_{H}1.

V_{L}1 will generally be joined directly to CL.

In one embodiment V_{H}1 C_{H}1 and V_{L}1 CL together form a Fab or Fab'.

In one embodiment an amino acid in V_{H}2 (for example the N-terminal thereof) is directly linked to an amino acid of C_{H}1 by a peptide bond (for example to the C-terminal of C_{H}1).

In one embodiment an amino acid in V_{H}2 (for example the N-terminal thereof) is linked to C_{H}1 indirectly by a linker (for example to the C-terminal of C_{H}1).

In one embodiment an amino acid in V_{L}2 (for example the N-terminal thereof) is directly linked to an amino acid of CL by a peptide bond (for example to the C-terminal of CL).

In one embodiment an amino acid in V_{L}2 (for example the N-terminal thereof) is linked to CL indirectly by a linker (for example to the C-terminal of CL).

In one embodiment V_{H}1 in the heavy chain is a variable domain from a heavy chain. That is to say is derived from the natural heavy chain of an antibody or relevant fragment thereof, or is derived from an alternative source such phage display and has the characteristics of a variable domain derived from a heavy chain.

In one embodiment V_{H}1 in the heavy chain is a variable domain from a light chain. That is to say is derived from the natural light chain of an antibody or relevant fragment thereof, or is derived from an alternative source such phage display and has the characteristics of a variable domain derived from a light chain.

In one embodiment V_{H}2 in the heavy chain is a variable domain from a heavy chain.

In one embodiment V_{H}2 in the heavy chain is a variable domain from a light chain.

In one embodiment V_{L}1 in the light chain is a variable domain from a heavy chain.

In one embodiment V_{L}1 in the light chain is a variable domain from a light chain.

In one embodiment V_{L}2 in the light chain is a variable domain from a heavy chain.

In one embodiment V_{L}2 in the light chain is a variable domain from a light chain.

In one embodiment V_{H}1 is a variable domain from a light chain and V_{H}2 is a variable domain from a light chain.

In one embodiment V_{H}1 is a variable domain from a heavy chain and V_{H}2 is a variable domain from a heavy chain.

In one embodiment V_{H}1 is a variable domain from a light chain and V_{H}2 is a variable domain from a heavy chain.

In one embodiment V_{H}1 is a variable domain from a heavy chain and V_{H}2 is a variable domain from a light chain.

In one embodiment V_{L}1 is a variable domain from a light chain and V_{L}2 is a variable domain from a light chain.

In one embodiment V_{L}1 is a variable domain from a heavy chain and V_{L}2 is a variable domain from a heavy chain.

In one embodiment V_{L}1 is a variable domain from a light chain and V_{L}2 is a variable domain from a heavy chain.

In one embodiment V_{L}1 is a variable domain from a heavy chain and V_{L}2 is a variable domain from a light chain.

In one embodiment the first variable domain pair bind the same epitope as the second variable domain pair.

In one embodiment the fusion protein of the invention avidly binds the target antigen.

In one embodiment the first variable domain pair bind the same antigen as the second variable domain pair, for example the first variable domain pair bind and the second variable domain pair bind different epitopes on the same antigen.

Thus in one embodiment the fusion protein according to the present disclosure is mono-specific. Monospecific as employed herein is intended to refer to the fact that all the binding sites bind the same target antigen.

In one aspect of this embodiment all the binding sites bind the same epitope(s) of said antigen.

In an alternative embodiment at least two binding sites bind different epitopes on the target antigen.

In one embodiment the first variable domain pair binds a different/distinct antigen to the second variable domain pair.

Thus in one embodiment the fusion protein according to the present disclosure is bispecific, for example the two binding sites specifically bind different or distinct antigens.

Specifically binds as employed herein is intended to refer to antibodies that have high affinity for a target antigen (i.e. antigens to which they are specific) and which bind antigens to which they are not specific with a low or much lower affinity (or not at all). Methods of measuring affinity are known to those skilled in the art and include such assays as BIAcore.

In one embodiment there is a disulfide bond between the variable domains which form a first binding site and further disulfide bond between the variable domains which form a second binding site.

The disulfide bond may be between the residues listed below (unless the context indicates otherwise Kabat numbering is employed in the list below). Wherever reference is made to Kabat numbering the relevant reference is Kabat et al., 1987, in Sequences of Proteins of Immunological Interest, US Department of Health and Human Services, NIH, USA):
- VH37 + VL95C see for example Protein Science 6, 781-788 Zhu et al (1997);
- VH44 + VL100 see for example; Biochemistry 33 5451-5459 Reiter et al (1994); or Journal of Biological Chemistry Vol. 269 No. 28 pp.18327-18331 Reiter et al (1994); or Protein Engineering, vol.10 no.12 pp.1453-1459 Rajagopal et al (1997);
- VH44 + VL105 see for example J Biochem. 118, 825-831 Luo et al (1995);
- VH45 + VL87 see for example Protein Science 6, 781-788 Zhu et al (1997);
- VH55 + VL101 see for example FEBS Letters 377 135-139 Young et al (1995);
- VH100 + VL50 see for example Biochemistry 29 1362-1367 Glockshuber et al (1990);
- VH100b + VL49;
- VH98 + VL 46 see for example Protein Science 6, 781-788 Zhu et al (1997);
- VH101 + VL46
- VH105 + VL43 see for example; Proc. Natl. Acad. Sci. USA Vol. 90 pp.7538-7542 Brinkmann et al (1993); or Proteins 19, 35-47 Jung et al (1994) or
- VH106 + VL57 see for example FEBS Letters 377 135-139 Young et al (1995).

The amino acid pairs listed above are in the positions conducive to replacement by cysteines such that disulfide bonds can be formed. Cysteines can be engineered into these positions by known techniques.

Accordingly a variable domain pair (VH/VL) may be linked by a disulfide bond between two cysteine residues, one in VH and one in VL, wherein the position of the pair of cysteine residues is selected from the group consisting of VH37 and VL95, VH44 and VL100, VH44 and VL105, VH45 and VL87, VH100 and VL50, VH100b and VL49, VH98 and VL46, VH101 and VL46, VH105 and VL43 and VH106 and VL57.

A variable domain pair (VH/VL) may be linked by a disulfide bond between two cysteine residues, one in VH and one in VL, which are outside of the CDRs wherein the position of the pair of cysteine residues is selected from the group consisting of VH37 and VL95, VH44 and VL100, VH44 and VL105, VH45 and VL87, VH100 and VL50, VH98 and VL46, VH105 and VL43 and VH106 and VL57.

A variable domain pair (VH/VL) may be linked by a disulfide bond between two cysteine residues, one in VH and one in VL, which are outside of the CDRs wherein the position of the pair of cysteine residues is selected from the group consisting of VH37 and VL95, VH44 and VL105, VH45 and VL87, VH100 and VL50, VH98 and VL46, VH105 and VL43 and VH106 and VL57.

In one embodiment a variable domain pair (VH/VL) of the present invention may be linked by a disulfide bond between two cysteine residues wherein the cysteine residue of VH is at position 44 and the cysteine residue of VL is at position 100.

Typically the cysteine pairs are engineered into those positions in VH and VL, accordingly a variable domain pair (VH/VL) may be linked by a disulfide bond between two engineered cysteine residues, one in VH and one in VL, wherein the position of the pair of engineered cysteine residues is selected from the group consisting of VH37 and VL95, VH44 and VL100, VH44 and VL105, VH45 and VL87, VH100 and VL50, VH100b and VL49, VH98 and VL46, VH101 and VL46, VH105 and VL43 and VH106 and VL57.

A variable domain pair (VH/VL) may be linked by a disulfide bond between two engineered cysteine residues, one in VH and one in VL, which are outside of the CDRs wherein the position of the pair of engineered cysteine residues is selected from the group consisting of VH37 and VL95, VH44 and VL100, VH44 and VL105, VH45 and VL87, VH100 and VL50, VH98 and VL46, VH105 and VL43 and VH106 and VL57.

A variable domain pair (VH/VL) may be linked by a disulfide bond between two engineered cysteine residues, one in VH and one in VL, which are outside of the CDRs wherein the position of the pair of engineered cysteine residues is selected from the group consisting of VH37 and VL95, VH44 and VL105, VH45 and VL87, VH100 and VL50, VH98 and VL46, VH105 and VL43 and VH106 and VL57.

In one embodiment the variable domain pair (VH/VL) is linked by a disulfide bond between two engineered cysteine residues wherein the engineered cysteine residue of VH is at position 44 and the engineered cysteine residue of VL is at position 100.

In one embodiment the V_{H}1 is fused directly to C_{H}1 and V_{L}1 is fused directly to CL and the there is a disulfide bond between the V_{H}2 and V_{L}2.

In one embodiment the one or more disulfide bonds between the variable regions of one or more binding sites, have a stabilizing effect and, for example, aid expression and/or minimises inappropriate dimerisation.

In one embodiment there is a disulfide bond between the variable domain(s) according to the present invention and a disulfide bond between the constant region fragments, such as C_{H}1 and CL.

Any of the formats provided herein may be provided with or without a disulfide bond between the constant domains.

The CL domain is derived from either Kappa or Lambda. In one embodiment CL is cKappa.

In one embodiment the "natural" disulfide bond is present between C_{H}1 and CL. The natural position for a bond forming 'interchain' cysteine is 214 in human cKappa and cLambda (Kabat numbering 4th edition 1987).

The exact location of the bond forming in cysteine, or 'interchain cysteine' in C_{H}1 depends on the particular domain actually employed. Thus, for example in human gamma-1 the natural position of the interchain cysteine forming the disulfide bond is located at position 233 (Kabat numbering 4th edition 1987). The position of the bond forming cysteine for other human isotypes such as gamma 2, 3, 4, IgM and IgD are known, for example 127.

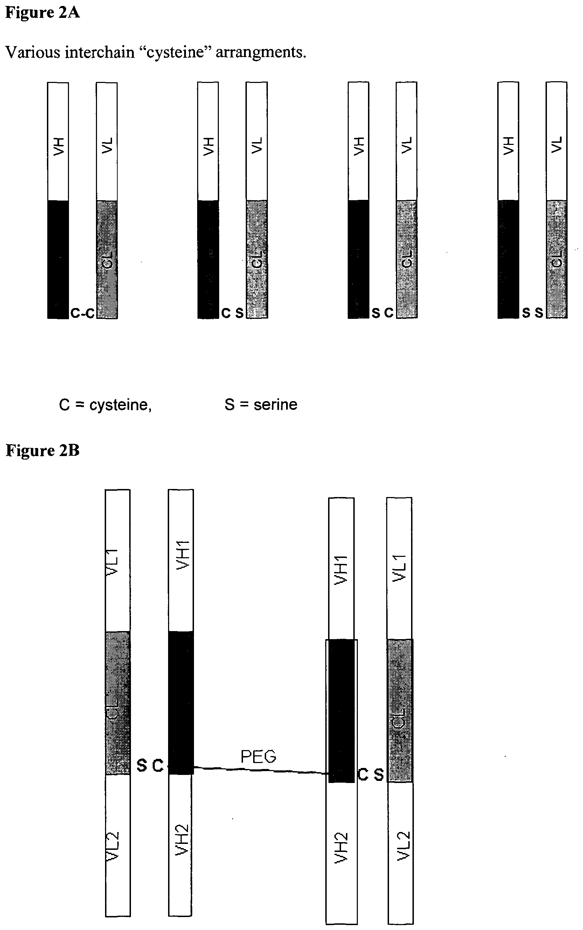

Various interchain disulfide bonds and lack thereof are shown in Fig 2A.

In one embodiment the fusion protein according to the disclosure has a disulfide bond in a position equivalent of corresponding to that in the naturally occurring C_{H}1 and CL.

In one embodiment constant region comprising C_{H}1 or CL has a disulfide bond which is in a non-naturally occurring position. This may be engineered into the molecule by introducing cysteine(s) into the amino acid chain at the positions required. This non-natural disulfide bond is in addition to or as an alternative to the natural disulfide bond present between C_{H}1 and CL.

In one embodiment the natural disulfide bond between C_{H}1 and CL is absent. In one embodiment all interchain disulfide bonds between C_{H}1 and CL are absent.

In one embodiment each constant region fragment is fused to at least one variable domain.

In one embodiment each constant region fragment is also linked via a peptide, for example an artificial/non-naturally occurring linker such as sequence in Table 1 and/or 2, to a variable domain, for example which is a non-cognate pair to the variable domain fused thereto.

In one embodiment the constant region fragment, for example in the heavy chain, comprises a C_{H}1 domain. In one embodiment the constant region fragment consists of a C_{H}1 domain.

The C_{H}1 may be derived from human IgA, IgD, IgE, IgG (such as IgG1, IgG2, IgG3, IgG4) or IgM domains and isotypes thereof.

In one embodiment the light chain comprises a CL domain. In one embodiment the constant region in the light chain consists of CL domain.

In one embodiment from the N-terminal the light chain is arranged as follows a V_{L}1 (part of a first cognate pair) a CL, a V_{L}2 (part of a second cognate pair), for example a CL may be fused to V_{L}1 of the first cognate pair and linked via a peptide to the V_{L}2 of the second cognate pair.

Examples of suitable peptide linkers are given below, for example in Table 1.

**Table1. Flexible linker seauences**

| **SEQ ID NO:** | **SEQUENCE** |
|---|---|
| 1 | SGGGGSE |
| 2 | DKTHTS |
| 3 | (S)GGGGS |
| 4 | (S)GGGGSGGGGS |
| 5 | (S)GGGGSGGGGSGGGGS |
| 6 | (S)GGGGSGGGGSGGGGSGGGGS |
| 7 | (S)GGGGSGGGGSGGGGSGGGGSGGGGS |
| 8 | AAAGSG-GASAS |
| 9 | AAAGSG-XGGGS-GASAS |
| 10 | AAAGSG-XGGGSXGGGS -GASAS |
| 11 | AAAGSG- XGGGSXGGGSXGGGS -GASAS |
| 12 | AAAGSG- XGGGSXGGGSXGGGSXGGGS-GASAS |
| 13 | AAAGSG-XS-GASAS |
| 14 | PGGNRGTTTTRRPATTTGSSPGPTQSHY |
| 15 | ATTTGSSPGPT |
| 16 | ATTTGS |
| 17 | GS |
| 18 | EPSGPISTINSPPSKESHKSP |
| 19 | GTVAAPSVFIFPPSD |
| 20 | GGGGIAPSMVGGGGS |
| 21 | GGGGKVEGAGGGGGS |
| 22 | GGGGSMKSHDGGGGS |
| 23 | GGGGNLITIVGGGGS |
| 24 | GGGGVVPSLPGGGGS |
| 25 | GGEKSIPGGGGS |
| 26 | RPLSYRPPFPFGFPSVRP |
| 27 | YPRSIYIRRRHPSPSLTT |
| 28 | TPSHLSHILPSFGLPTFN |
| 29 | RPVSPFTFPRLSNSWLPA |
| 30 | SPAAHFPRSIPRPGPIRT |
| 31 | APGPSAPSHRSLPSRAFG |
| 32 | PRNSIHFLHPLLVAPLGA |
| 33 | MPSLSGVLQVRYLSPPDL |
| 34 | SPQYPSPLTLTLPPHPSL |
| 35 | NPSLNPPSYLHRAPSRIS |
| 36 | LPWRTSLLPSLPLRRRP |
| 37 | PPLFAKGPVGLLSRSFPP |
| 38 | VPPAPVVSLRSAHARPPY |
| 39 | LRPTPPRVRSYTCCPTP- |
| 40 | PNVAHVLPLLTVPWDNLR |
| 41 | CNPLLPLCARSPAVRTFP |

(S) is optional in sequences 3 to 7. The linkers employed in the present disclosure may comprise a linker shown in Table 1.

Examples of rigid linkers, for use in fusion proteins according to the present disclosure include, the peptide sequences GAPAPAAPAPA (SEQ ID NO:42), PPPP (SEQ ID NO:43) and PPP.

In one embodiment the peptide linker comprises an albumin binding peptide.

Examples of albumin binding peptides are provided in WO 2007/106120 and include:

**Table 2**

| **SEQ ID NO:** | **SEQUENCE** |
|---|---|
| 45 | DLCLRDWGCLW |
| 46 | DICLPRWGCLW |
| 47 | MEDICLPRWGCLWGD |
| 48 | QRLMEDICLPRWGCLWEDDE |
| 49 | QGLIGDICLPRWGCLWGRSV |
| 50 | QGLIGDICLPRWGCLWGRSVK |
| 51 | EDICLPRWGCLWEDD |
| 52 | RLMEDICLPRWGCLWEDD |
| 53 | MEDICLPRWGCLWEDD |
| 54 | MEDICLPRWGCLWED |
| 55 | RLMEDICLARWGCLWEDD |
| 56 | EVRSFCTRWPAEKSCKPLRG |
| 57 | RAPESFVCYWETICFERSEQ |
| 58 | EMCYFPGICWM |

In one embodiment the linker has an effector function.

In one embodiment the fusion proteins of the disclosure are dimerised, to provide a molecule referred to herein as (Fab'Fv)₂. Dimerisation for example may be via formation of a disulphide bond between two solvent accessible 'target' cysteines on each of two fusion protein according to the disclosure (i.e Fab-dsFv), or chemical cross-linking of 'target' cysteines, lysines, sugar moieties or un-natural amino acids on each of two Fab-Fv.

Where formation of inter-Fab-Fv disulphide bond formation (intermolecular bond formation) is desired only one linker within a Fab-Fv contains cysteine or cysteines. That is to say that when the linker between CL and V_{L}2 does contain one or more cysteines, the linker between C_{H}1 and V_{H}2 will usually be devoid of cysteines.

When the linker between C_{H}1 and V_{H}2 contains one or more cysteines, then the linker between CL and V_{L}2 will usually be devoid of cysteines.

Linkers may contain between 1 and 8 cysteines and may be composed of any peptide sequence containing 1 to 8 cysteines.

Suitable peptide sequences shown in Table 1, 2 and 3 and these may also be engineered to contain 1 to 8 cysteines, as required.

Hinges may be employed as linkers to promote dimerisation, since they are naturally flexible and contain sufficient secondary structure as to promote efficient and stable disulphide formation. In particular natural hinges or modified hinges of any class or isotype may be used when they contain between 1 and 8 cysteine residues.

When a natural or modified hinge linker sequence between CL and V_{L}2 does contain one or more cysteines then the linker between C_{H}1 and V_{H}2 will usually be devoid of cysteines.

When the hinge linker between C_{H}1 and V_{H}2 does contain one or more cysteines, then the linker between CL and V_{L}2 is usually devoid of cysteines.

Alternatively, inter- Fab-Fv dimerisation may be promoted by the engineering of 'target' residues on any solvent or surface accessible area of V_{L}1, V_{H}1, cKappa, cLambda, C_{H}1, V_{L}2 or V_{H}2. Similarly, 'target' residues may be engineered to be at the C-terminus of either light chain or heavy chain polypeptides, for example after V_{L}2 of after V_{H}2. These target residues, will preferably be cysteine residues immediately after the last residues of V_{L}2 or V_{H}2 or in a linker, hinge or spacer region of polypeptide.

Where 'target' residues are linked by a chemical cross-linker, the length, composition, activity or hetero-functionality may be varied in order to fine tune antigen accessibility, functional avidity, serum half-life, purification properties or storage and formulation properties. The chemical cross-linker may be composed in part of polyethylene glycol, or may contain additional reactive groups for addition of a third specificity or active agent.

In one embodiment a fusion protein of the present invention is dimerised using a polymer, for example PEG, such that one chain in a first fusion protein is conjugated to a polypeptide, for example an antibody or fragment to form a heterodimer.

In one embodiment a fusion protein of the present invention is dimerised using a polymer, to a second fusion protein according to the present disclosure to form a heterodimer or a homodimer.

Figure 2B shows an example of a PEG linked dimer format comprising two proteins of the present disclosure. The PEG linker is linked between C_{H}1 in one fusion protein and C_{H}1 of the second fusion protein. However, a hinge region may be incorporated with suitable properties for conjugating a PEG linker molecule thereto. Alternatively a CL domain may be employed to conjugate the PEG thereto.

The position of the disulfide bond between one or more variable domain pairs can be in any of the positions, described herein.

The format may also be provided with or without a disulfide bond between the constant regions, examples of which are discussed above in more detail.

Hinges may be natural hinges or modified hinges. Modified hinges may be employed as per Table 3.

A number of modified hinge regions have already been described for example, in US5,677,425, US6642356, WO9915549, WO2005003170, WO2005003169, WO2005003170, WO9825971 and WO2005003171 and these are incorporated herein by reference. Particular examples of hinges include those shown in Table 3.

**Table 3. Hinge linker sequences**

| **SEQ ID NO:** | **SEQUENCE** |
|---|---|
| 59 | DKTHTCXX |
| 60 | DKTHTCPPCPA |
| 61 | DKTHTCPPCPATCPPCPA |
| 62 | DKTHTCPPCPATCPPCPATCPPCPA |
| 63 | DKTHTCPPCPAGKPTLYNSLVMSDTAGTCY |
| 64 | DKTHTCPPCPAGKPTHVNVSVVMAEVDGTCY |
| 65 | DKTHTCCVECPPCPA |
| 66 | DKTHTCPRCPEPKSCDTPPPCPRCPA |
| 67 | DKTHTCPSCPA |
| 68 | DKTHTSXX |
| 69 | DKTHTCPPSPA |
| 70 | DKTHTSPPCPA |
| 71 | DKTHTSPPSPA |
| 72 | DKTHTCPPCPATCPPSPA |
| 73 | DKTHTCPPCPATSPPCPA |
| 74 | DKTHTCPPSPATCPPCPA |
| 75 | DKTHTSPPCPATCPPCPA |
| 76 | DKTHTCPPCPATSPPSPA |
| 77 | DKTHTCPPSPATCPPSPA |
| 78 | DKTHTSPPCPATCPPSPA |
| 79 | DKTHTSPPSPATCPPCPA |
| 80 | DKTHTSPPCPATSPPCPA |
| 81 | DKTHTCPPSPATSPPCPA |
| 82 | DKTHTSPPSPATSPPSPA |
| 83 | DKTHTCPPSPATSPPSPA |
| 84 | DKTHTSPPCPATSPPSPA |
| 85 | DKTHTSPPSPATCPPSPA |
| 86 | DKTHTSPPSPATSPPCPA |
| 87 | DKTHTCPPCPATCPPCPATCPPSPA |
| 88 | DKTHTCPPCPATCPPCPATSPPCPA |
| 89 | DKTHTCPPCPATCPPSPATCPPCPA |
| 90 | DKTHTCPPCPATSPPCPATCPPCPA |
| 91 | DKTHTCPPSPATCPPCPATCPPCPA |
| 92 | DKTHTSPPCPATCPPCPATCPPCPA |
| 93 | DKTHTCPPCPATCPPCPATSPPSPA |
| 94 | DKTHTCPPCPATCPPSPATCPPSPA |
| 95 | DKTHTCPPCPATSPPCPATCPPSPA |
| 96 | DKTHTCPPSPATCPPCPATCPPSPA |
| 97 | DKTHTSPPCPATCPPCPATCPPSPA |
| 98 | DKTHTCPPCPATCPPSPATSPPCPA |
| 99 | DKTHTCPPCPATSPPCPATSPPCPA |
| 100 | DKTHTCPPSPATCPPCPATSPPCPA |
| 101 | DKTHTSPPCPATCPPCPATSPPCPA |
| 102 | DKTHTCPPCPATSPPSPATCPPCPA |
| 103 | DKTHTCPPSPATCPPSPATCPPCPA |
| 104 | DKTHTSPPCPATCPPSPATCPPCPA |
| 105 | DKTHTCPPSPATSPPCPATCPPCPA |
| 106 | DKTHTSPPCPATSPPCPATCPPCPA |
| 107 | DKTHTSPPSPATCPPCPATCPPCPA |
| 108 | DKTHTCPPCPATCPPSPATSPPSPA |
| 109 | DKTHTCPPCPATSPPCPATSPPSPA |
| 110 | DKTHTCPPSPATCPPCPATSPPSPA |
| 111 | DKTHTSPPCPATCPPCPATSPPSPA |
| 112 | DKTHTCPPCPATSPPSPATCPPSPA |
| 113 | DKTHTCPPSPATCPPSPATCPPSPA |
| 114 | DKTHTSPPCPATCPPSPATCPPSPA |
| 115 | DKTHTCPPSPATSPPCPATCPPSPA |
| 116 | DKTHTSPPCPATSPPCPATCPPSPA |
| 117 | DKTHTSPPSPATSPPCPATCPPCPA |
| 118 | DKTHTSPPSPATCPPSPATCPPCPA |
| 119 | DKTHTSPPSPATCPPCPATSPPCPA |
| 120 | DKTHTSPPSPATCPPCPATCPPSPA |
| 121 | DKTHTCPPSPATSPPSPATCPPCPA |
| 122 | DKTHTCPPSPATSPPCPATSPPCPA |
| 123 | DKTHTCPPSPATCPPSPATSPPSPA |
| 124 | DKTHTCPPSPATSPPSPATCPPSPA |
| 125 | DKTHTSPPSPATSPPCPATCPPSPA |
| 126 | DKTHTSPPSPATSPPSPATCPPCPA |
| 127 | DKTHTSPPSPATSPPCPATSPPCPA |
| 128 | DKTHTSPPSPATSPPCPATCPPSPA |
| 129 | DKTHTCPPSPATSPPSPATSPPCPA |
| 130 | DKTHTCPPSPATSPPSPATSPPSPA |
| 131 | DKTHTSPPSPATSPPSPATSPPCPA |
| 132 | DKTHTSPPCPATSPPSPATSPPSPA |
| 133 | DKTHTSPPSPATCPPSPATSPPSPA |
| 134 | DKTHTSPPSPATSPPCPATSPPSPA |
| 135 | DKTHTSPPSPATSPPSPATCPPSPA |
| 136 | DKTHTSPPSPATSPPSPATSPPSPA |
| 137 | DKTHTCPPCPAGKPTLYNSLVMSDTAGTSY |
| 138 | DKTHTCPPSPAGKPTLYNSLVMSDTAGTCY |
| 139 | DKTHTSPPCPAGKPTLYNSLVMSDTAGTCY |
| 140 | DKTHTCPPSPAGKPTLYNSLVMSDTAGTSY |
| 141 | DKTHTSPPSPAGKPTLYNSLVMSDTAGTCY |
| 142 | DKTHTSPPCPAGKPTLYNSLVMSDTAGTSY |
| 143 | DKTHTSPPSPAGKPTLYNSLVMSDTAGTSY |
| 1441 | DKTHTCPPCPAGKPTHVNVSVVMAEVDGTSY |
| 145 | DKTHTCPPSPAGKPTHVNVSVVMAEVDGTCY |
| 146 | DKTHTSPPCPAGKPTHVNVSVVMAEVDGTCY |
| 147 | DKTHTCPPSPAGKPTHVNVSVVMAEVDGTSY |
| 148 | DKTHTSPPCPAGKPTHVNVSVVMAEVDGTSY |
| 149 | DKTHTSPPSPAGKPTHVNVSVVMAEVDGTCY |
| 150 | DKTHTSPPSPAGKPTHVNVSVVMAEVDGTSY |
| 151 | DKTHTCCVECPPSPA |
| 152 | DKTHTCCVESPPCPA |
| 153 | DKTHTCSVECPPCPA |
| 154 | DKTHTSCVECPPCPA |
| 155 | DKTHTCCVESPPSPA |
| 156 | DKTHTCSVECPPSPA |
| 157 | DKTHTSCVECPPSPA |
| 158 | DKTHTCSVESPPCPA |
| 159 | DKTHTSSVECPPCPA |
| 160 | DKTHTCSVESPPSPA |
| 161 | DKTHTSSVECPPSPA |
| 162 | DKTHTSSVESPPCPA |
| 163 | DKTHTSSVESPPSPA |
| 164 | DKTHTCPRCPEPKSCDTPPPCPRSPA |
| 165 | DKTHTCPRCPEPKSCDTPPPSPRCPA |
| 166 | DKTHTCPRCPEPKSSDTPPPCPRCPA |
| 167 | DKTHTCPRSPEPKSCDTPPPCPRCPA |
| 168 | DKTHTSPRCPEPKSCDTPPPCPRCPA |
| 169 | DKTHTCPRCPEPKSCDTPPPSPRSPA |
| 170 | DKTHTCPRCPEPKSSDTPPPCPRSPA |
| 171 | DKTHTCPRSPEPKSCDTPPPCPRSPA |
| 172 | DKTHTSPRCPEPKSCDTPPPCPRSPA |
| 173 | DKTHTCPRCPEPKSSDTPPPSPRSPA |
| 174 | DKTHTCPRSPEPKSCDTPPPSPRSPA |
| 175 | DKTHTSPRCPEPKSCDTPPPSPRSPA |
| 176 | DKTHTCPRSPEPKSSDTPPPCPRSPA |
| 177 | DKTHTSPRCPEPKSSDTPPPCPRSPA |
| 178 | DKTHTSPRSPEPKSCDTPPPCPRSPA |
| 179 | DKTHTCPRSPEPKSSDTPPPSPRCPA |
| 180 | DKTHTSPRCPEPKSSDTPPPSPRCPA |
| 181 | DKTHTSPRSPEPKSSDTPPPCPRCPA |
| 182 | DKTHTCPRSPEPKSSDTPPPSPRSPA |
| 183 | DKTHTSPRSPEPKSSDTPPPCPRSPA |
| 184 | DKTHTSPRSPEPKSCDTPPPSPRSPA |
| 185 | DKTHTSPRCPEPKSSDTPPPSPRSPA |
| 186 | DKTHTSPRSPEPKSSDTPPPSPRSPA |
| 187 | DKTHTCPSSPA |
| 188 | DKTHTSPSCPA |
| 189 | DKTHTSPSSPA |

wherein in X represents any amino acid, for example XX may represent AA.

In one embodiment the fusion protein according to the present disclosure does not comprise a hinge.

The inventors believe that by providing variable domains as cognate pairs in the final construct optimises and maintains the antigen bind properties of the binding site formed by the relevant pair.

The disulfide bridges in the cognate pairs are believed to be advantageous in that they assist in stabilizing the format.

It will be appreciated that one or more amino acid substitutions, additions and/or deletions may be made to the antibody variable domains, provided by the present invention, without significantly altering the ability of the antibody to bind to target antigen and to neutralise activity thereof. The effect of any amino acid substitutions, additions and/or deletions can be readily tested by one skilled in the art, for example by using the *in vitro* assays, for example a BIAcore assay.

In one embodiment the interchain disulfide bond between the light chain and heavy chain is not present and one or two of the cysteines that would not normally form the bond (interchain cysteines) are conjugated to a polymer. One interchain cysteine can be selectively conjugated employing genetic engineering techniques to replace the corresponding interchain cysteine with an alternative amino acid such as serine.

Both interchain cysteines may be conjugated to a polymer by employing suitably strong reducing agents. Various arrangements of interchain cysteines or lack of them are represented in Figure 2A.

The kappa or lambda domain may be modified by chemical conjugation with a suitable polymer.

In one embodiment a human cKappa in a fusion protein of the present disclosure comprises at the C-terminal a sequence selected from:
SFNRGEC (SEQ ID NO:190);
SFNRGCS (SEQ ID NO:191);
SFNRCES (SEQ ID NO:192);
SFNCGES (SEQ ID NO:193);
SFCRGES (SEQ ID NO:194); and
SCNRGES (SEQ ID NO:195).

One or more cysteines in the C-terminal of cKappa, for example as described directly above, may be used to conjugate a PEG polymer thereto.

Suitable positions in cKappa for conjugation include:
1. **Upper cKappa,** which is approximately equidistant from V_{L}1 and V_{L}2 in a fusion protein according to the present disclosure, for example Glu143, Gln199 and/or Val110 (linear and Kabat numbering),
2. **Middle cKappa** which is distant from all mobile and flexible motifs which might be required for effective antigen binding, for example Lys145 and/or Gln147, (linear and Kabat numbering) and/or
3. **Lower cKappa,** for example Lys190, Asn210, Arg211, and/or Glu213 (linear and Kabat numbering).

These amino acids may be replaced, for example with cysteine, as required, employing known techniques.

In one embodiment the fusion protein comprises the sequence: 


(SEQ ID NO:196)
 for example in cKappa, in particular in the position 108-214 (Kabat numbering) in the amino acid sequence.

In one embodiment a human C_{H}1 in a fusion protein of the present disclosure comprises at the C-terminal a sequence selected from:
KSC,
KSS, and
KCS.

In one embodiment the fusion protein comprises the sequence: 
S120ASTKGPSVFPLAPSSKSTS139GGTAALGCLVKDYFPEPVTVSWNS163GALTSGV
HTFPAVLQSSGLYSLSSVVTVPSSSLGTQTYICNVNHKPSNTKVDKKV EPKSC222
(SEQ ID NO:197)
 Amino acid S¹²⁰ shown in bold is not part of the genetic / exon definition of C_{H}1, but is considered to be part of the strucural 'elbow'
Underlined sequence shown is not part of the genetic / exon definition of C_{H}1, but is part of upper hinge
for example in C_{H}1, in particular in the position 121-218 (Kabat numbering) in the amino acid sequence.

Suitable positions in C_{H}1 for conjugation include:
1. **Upper C_{H}1** which is approximately equidistant from V_{H}1 and V_{H}2 in fusion proteins according to the present invention, for example Asn211 (216) and/or Thr123 (Asn 216 and/or Thr 116 by Kabat numbering),
2. **Middle C_{H}1** which is distant from all mobile and flexible motifs which might be required for effective antigen binding, for example Ser163 and/or Ser127 (Ser163 and/or Ser120 by Kabat numbering), and/or
3. **Lower C_{H}1** for example Lys136, Ser137, and/or Ser222 (Lys129, Ser130, Ser232 by Kabat numbering).

These amino acids may be replaced, for example with cysteine, as required employing known techniques. In one embodiment therefore an engineered cysteine according to the present invention refers to where the naturally occurring residue at a given amino acid position has been replaced with a cysteine residue.

Introduction of engineered cysteines can be performed using any method known in the art. These methods include, but are not limited to, PCR extension overlap mutagenesis, site-directed mutagenesis or cassette mutagenesis (see, generally, Sambrook et al., Molecular Cloning, A Laboratory Manual, Cold Spring Harbour Laboratory Press, Cold Spring Harbour, NY, 1989; Ausbel et al., Current Protocols in Molecular Biology, Greene Publishing & Wiley-Interscience, NY, 1993). Site-directed mutagenesis kits are commercially available, e.g. QuikChange® Site-Directed Mutagenesis kit (Stratagen, La Jolla, CA). Cassette mutagenesis can be performed based on Wells et al., 1985, Gene, 34:315-323. Alternatively, mutants can be made by total gene synthesis by annealing, ligation and PCR amplification and cloning of overlapping oligonucleotides.

In one embodiment the fusion protein has no cysteines in the CH1 and/or CL regions for conjugation to a polymer.

In one embodiment VH1 is fused to CH1 and the amino acid sequence connecting the same is referred to herein as the elbow. In one embodiment said elbow has one or more, such as one, PEG polymers conjugated thereto, for example conjugated at a position equidistant between VH1 and VH2 such as conjugated to Thr123 (Thr 116 by Kabat numbering) and/or S119 (Ser 112 by Kabat numbering) and/or S122 (Ser115 by Kabat numbering) residues. The disulfide bond is provided, for example by engineering a cysteine or cysteines into suitable positions in the molecule to provide a substrate for conjugating a PEG polymer thereto.

In one embodiment VL1 is fused to CL and the amino acid sequence connecting the same is referred to herein as the elbow. In one embodiment said elbow has one or more, such as one, PEG polymer conjugated thereto for example conjugated at a position equidistant between VL1 and VL2 such as conjugated to Lys107, Arg108 and/or Thr109 (linear and Kabat numbering) residues. As discussed above cysteines can be provided as required for conjugation.

In one embodiment the PEG is conjugated to a residue that is spatially close to areas of aggregation propensity. Advantageously, PEGylation close to such areas may substantially mask the region of interest from inter-molecular interactions at concentrations in solution suitable for therapeutic doses. In the light chain the conjugation may for example be at or about Thr109 or Gln199 (linear and Kabat numbering), for example to negate the ValAla-AlaLys spot, and/or Lys149 or Asn152 (linear and Kabat numbering), for example to negate the single Leu spot. In the heavy chain the conjugation may, for example be at or about Gln178 (Gln 179 by Kabat numbering) or Ser180 (Ser 182 by Kabat numbering) or Gly181 (Gly 183 by Kabat numbering) to deal with the ValLys-Tyr spot.

In the molecules of the present invention the PEG molecule is attached to a solvent accessible, reactive and/or surface exposed/accessible amino acid such as cysteine.
A surface exposed cysteine (free cysteine) as employed herein is intended to refer to cysteine that when the fusion protein in a "natural" folded conformation is accessible for conjugating an effector molecule, such as a PEG molecule, thereto. A surface cysteine is one found in a hydrophilic part of the antibody or fragment. Examples of how to engineer free cysteines of this type are also provided in US7,521,541.

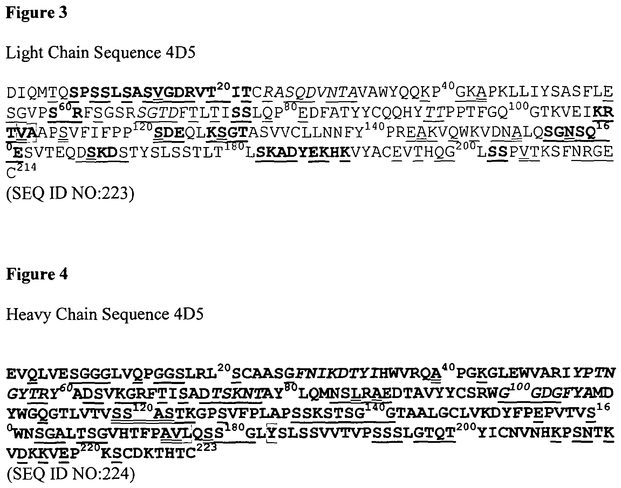

Suitable amino acids in the light chain of 4D5 (sequence shown in Fig 3), which may be replaced by cysteine include:
Serine (S): 7,9, 10, 12, 14, 26, 56, 60, 63, 67, 76, 77, 114, 121, 127, 156, 159, 168, 171,202,203,
Threonine (T): 5,20, 22, 31, 69, 72, 74, 129, 197, 206,
Glycine (G): 16, 41, 57, 68, 128, 143, 157, 200, 212
Aspartate (D): 17, 28, 70, 122, 151, 167, 170
Arginine (R): 18, 24, 61, 211,
Glutamine (Q): 27, 79, 147, 160, 195, 199
Asparagine (N): 30, 152, 158, 210,
Alanine (A): 34, 153,
Lysine (K): 39, 42, 126, 145, 149, 169,
Glutamate (E): 55, 81, 123, 161, 213.

The numbers employed above are the antibody primary sequence numbering as shown in PDB crystal structure sequence file, 1FVE. These numbers also correspond to Kabat numbering. The disclosure also extends to replacement of corresponding amino acids (employing Kabat numbering) in other antibodies or fragments comprised in fusion proteins of the invention.

Accordingly in one embodiment the antibody fusion protein light chain comprises an engineered cysteine wherein the position of said engineered cysteine is selected from the group consisting of S7, S9, S10, S12, S14, S26, S56, S60, S63, S67, S76, S77, S114, S121, S127, S156, S159, S168, S171, S202, S203, T5, T20, T22, T31, T69, T72, T74, T129, T197, T206, G16, G41, G57, G68, G128, G143, G157, G200, G212, D17, D28, D70, D122, D151, D167, D170, R18, R24, R61, R211, Q27, Q79, Q147, Q160, Q195, Q199, N30, N152, N158, N210, A34, A153, K39, K42, K126, K145, K149, K169, E55, E81, E123, E161 and E213.

In one embodiment the antibody fusion protein light chain comprises an engineered cysteine wherein the position of said engineered cysteine is selected from the group consisting of S7, S9, S10, S12, S14, S26, S56, S60, S63, S67, S76, S77, S 114, S121, S127, S 156, S 159, S168, S171, S202, S203, T5, T20, T22, T31, T69, T72, T74, T109, T129, T197, T206, G16, G41, G57, G68, G128, G143, G157, G200, G212, D17, D28, D70, D122, D151, D167, D170, R18, R24, R61, R108, R211, Q27, Q79, Q147, Q160, Q195, Q199, N30, N152, N158, N210, A34, A153, K39, K42, K107, K126, K145, K149, K169, K190, E55, E81, E123, E143, E161 and E213.

Other suitable residues have been described in the literature including WO2006/034488 and WO2008/038024. Accordingly, in one embodiment a cysteine is engineered into the fusion protein light chain, for example:
V15 is replaced by C,
A43 is replaced by C,
V110 is replaced by C,
S 114 is replaced by C,
S121 is replaced by C,
S 127 is replaced by C,
A144 is replaced by C,
A153 is replaced by C,
N158 is replaced by C,
S 168 is replaced by C,
V205 is replaced by C,
S 171 is replaced by C,
S156 is replaced by C,
S202 is replaced by C, and/or
S203 is replaced by C.

The numbering above is by reference to the light chain sequence shown in Figure 3, but the disclosure herein also extends to corresponding Kabat positions in other light chains.

In one embodiment a cysteine is engineered into the fusion protein light chain, for example:
S171 is replaced by C,
S156 is replaced by C,
S202 is replaced by C, and/or
S203 is replaced by C.

In one embodiment the antibody fusion protein light chain comprises an engineered cysteine wherein the position of said engineered cysteine is selected from the group consisting of S7, S9, S10, S12, S14, S26, S56, S60, S63, S67, S76, S77, S 114, S121, S127, S 156, S 159, S168, S171, S202, S203, T5, T20, T22, T31, T69, T72, T74, T109, T129, T197, T206, V15, V110, V205, G16, G41, G57, G68, G128, G143, G157, G200, G212, D17, D28, D70, D122, D151, D167, D170, R18, R24, R61, R108, R211, Q27, Q79, Q147, Q160, Q195, Q199, N30, N152, N158, N210, A34, A43, A144, A153, K39, K42, K107, K126, K145, K149, K169, K190, E55, E81, E123, E143, E161 and E213.

In one embodiment the antibody fusion protein light chain comprises an engineered cysteine wherein the position of said engineered cysteine is selected from the group consisting of S7, S9, S10, S12, S14, S26, S56, S60, S63, S67, S76, S77, S159, T5, T20, T22, T31, T69, T72, T74, T109, T129, T197, T206, G16, G41, G57, G68, G128, G143, G157, G200, G212, D17, D28, D70, D122, D151, D167, D170, R18, R24, R61, R108, R211, Q27, Q79, Q147, Q160, Q195, Q199, N30, N152, N210, A34, K39, K42, K107, K126, K145, K149, K169, K190, E55, E81, E123, E143, E161 and E213.

In one embodiment a cysteine is engineered into the fusion protein light chain constant region, CL, for example:
T109 is replaced by C,
E143 is replaced by C,
K145 is replaced by C,
K149 is replaced by C and/or
N210 is replaced by C.

In one embodiment a cysteine is engineered into the fusion protein light chain variable region, for example:
S77 of SEQ ID NO:44 is replaced by C and/or
K107 of SEQ ID NI:44 is replaced by C.

In one embodiment the antibody fusion protein light chain comprises an engineered cysteine wherein the position of said engineered cysteine is selected from the group consisting of S77, K107, T109, E143, K145, K149 and N210.

All the residues specified herein above are derived from IgG1 but the disclosure herein also extends to corresponding Kabat positions in other light chains from other classes and isotypes of antibodies.

Accordingly, in one embodiment the antibody fusion protein light chain comprises an engineered cysteine wherein the position of said engineered cysteine is selected from the group consisting of 5, 7, 9, 10, 12, 14, 15, 16, 17, 18, 20, 22, 24, 26, 27, 28, 30, 31, 34, 39, 41, 42, 43, 55, 56, 57, 60, 61, 63, 67, 68, 69, 70, 72, 74, 76, 77, 79, 81, 107, 108, 109, 110, 114, 121, 122, 123, 126, 127, 128, 129, 143, 144, 145, 147, 149, 151, 152, 153, 156, 157, 158, 159, 160, 161, 167, 168, 169, 170, 171, 190, 195, 197, 199, 200, 202, 203, 205, 206, 210, 211, 212 and 213 numbered according to the Kabat numbering system.

In one embodiment the antibody fusion protein light chain comprises an engineered cysteine wherein the position of said engineered cysteine is selected from the group consisting of 5, 7, 9, 10, 12, 14, 16, 17, 18, 20, 22, 24, 26, 27, 28, 30, 31,34, 39, 41, 42, 55, 56, 57, 60, 61, 63, 67, 68, 69, 70, 72, 74, 76, 77, 79, 81, 107, 108, 109, 122, 123, 126, 128, 129, 143, 145, 147, 149, 151, 152, 157, 159, 160, 161, 167, 169, 170, 190, 195, 197, 199, 200, 206, 210, 211, 212 and 213 numbered according to the Kabat numbering system.

Accordingly, in one embodiment the antibody fusion protein light chain comprises an engineered cysteine wherein the position of said engineered cysteine is selected from the group consisting of position 77, 107, 109, 143, 145, 149 and 210 numbered according to the Kabat numbering system.

Suitable amino acids in the heavy chain of 4D5 (sequence shown in Fig 4), which may be replaced by cysteine include:
Serine (S): 7, 17, 21, 63, 71, 86, 119, 120, 122, 127, 134, 135, 137, 139, 160, 163, 168,179,180,193,194,195,210,222
Threonine (T): 58, 69, 123, 138, 167, 198, 200,
Glycine (G): 8, 9, 10, 15, 16, 26, 66, 100, 101, 103, 140, 141, 164, 169, 181, 197,
Aspartate (D): 62, 73, 102, 215,
Arginine (R): 59, 67, 87,
Glutamine (Q): 3, 13, 112, 155, 199, 219,
Asparagine (N): 84, 211,
Alanine (A): 88, 121, 165
Lysine (K): 43, 65, 124, 136, 208, 213, 217,
Glutamate (E): 89
The numbers employed above are the antibody primary sequence numbering as shown in PDB crystal structure sequence file, 1FVE. The disclosure also extends to replacement of corresponding amino acids (employing Kabat numbering) in other antibodies or fragments comprised in fusion proteins of the invention.

The equivalent Kabat sequence numbering for the positions in the heavy chain is:
Serine (S): 7, 17, 21, 62, 70, 82b, 112, 113, 115, 120, 127, 128, 130, 134, 156, 163, 168, 180, 182, 195, 196, 197, 215, 232
Threonine (T): 57, 68, 116, 133, 167, 200, 205,
Glycine (G): 8, 9, 10, 15, 16, 26, 65, 96, 97, 99, 135, 136, 164, 169, 183, 199,
Aspartate (D): 61, 72, 98, 220,
Arginine (R): 58, 66, 86,
Glutamine (Q): 3, 13, 105, 203
Asparagine (N): 82a, 216,
Alanine (A): 84, 114, 165
Lysine (K): 43, 64, 117, 129, 213, 218, 222,
Glutamate (E): 85
Accordingly in one embodiment the antibody fusion protein heavy chain comprises an engineered cysteine wherein the position of said engineered cysteine is selected from the group consisting of S7, S17, S21, S62, S70, S82b, S112, S113, S115, S120, S127, S128, S130, S134, S156, S163, S168, S180, S182, S195, S196, S197, S215, S232, T57, T68, T116, T133, T167, T200, T205, G8, G9, G10, G15, G16, G26, G65, G96, G97, G99, G135, G136, G164, G169, G183, G199, D61, D72, D98, D220, R58, R66, R86, Q3, Q13, Q105, Q203, N82a, N216, A84, A114, A165, K43, K64, K117, K129, K213, K218, K222 and E85.

In one embodiment the antibody fusion protein heavy chain comprises an engineered cysteine wherein the position of said engineered cysteine is selected from the group consisting of S7, S17, S21, S62, S70, S82b, S112, S113, S115, S120, S127, S128, S130, S134, S156, S163, S168, S180, S182, S195, S196, S197, S215, S232, T57, T68, T116, T133, T167, T200, T205, G8, G9, G10, G15, G16, G26, G65, G96, G97, G99, G135, G136, G164, G169, G183, G199, D61, D72, D98, D220, R58, R66, R86, Q3, Q13, Q105, Q179, Q203, N82a, N216, A84, A114, A165, K43, K64, K117, K129, K213, K218, K222 and E85.

Other suitable residues have been described in the literature including WO2006/034488 and WO2008/038024. Accordingly, in one embodiment a cysteine is engineered into the fusion protein heavy chain, for example:
A40 is replaced by C,
L86 is replaced by C,
A88 is replaced by C,
S119 is replaced by C,
S120 is replaced by C,
A121 is replaced by C,
S 122 is replaced by C,
A175 is replaced by C,
V176 is replaced by C, and/or
S179 is replaced by C.

The numbering above is by reference to the heavy chain sequence shown in Figure 4, but the disclosure herein also extends to corresponding Kabat positions in other heavy chains.

The equivalent positions by Kabat numbering are as follows:
A40 is replaced by C,
L82c is replaced by C,
A84 is replaced by C,
S112 is replaced by C,
S 113 is replaced by C,
A114 is replaced by C,
S115 is replaced by C,
A176 is replaced by C,
V177 is replaced by C, and/or
S180 is replaced by C.

Accordingly in one embodiment the antibody fusion protein heavy chain comprises an engineered cysteine wherein the position of said engineered cysteine is selected from the group consisting of S7, S17, S21, S62, S70, S82b, S112, S113, S115, S120, S127, S128, S130, S134, S156, S163, S168, S180, S182, S195, S196, S197, S215, S232, T57, T68, T116, T133, T167, T200, T205, G8, G9, G10, G15, G16, G26, G65, G96, G97, G99, G135, G136, G164, G169, G183, G199, D61, D72, D98, D220, R58, R66, R86, Q3, Q13, Q105, Q179, Q203, N82a, N216, A40, A84, A114, A165, A176, V177, L82c, K43, K64, K117, K129, K213, K218, K222 and E85.

In one embodiment the antibody fusion protein heavy chain comprises an engineered cysteine wherein the position of said engineered cysteine is selected from the group consisting of S7, S17, S21, S62, S70, S82b, S120, S127, S128, S130, S134, S156, S163, S168, S182, S195, S196, S197, S215, S232, T57, T68, T116, T133, T167, T200, T205, G8, G9, G10, G15, G16, G26, G65, G96, G97, G99, G135, G136, G164, G169, G183, G199, D61, D72, D98, D220, R58, R66, R86, Q3, Q13, Q105, Q179, Q203, N82a, N216, A165, K43, K64, K117, K129, K213, K218, K222 and E85.

In one embodiment a cysteine is engineered into the fusion protein heavy chain constant region CH1, for example:
T116 is replaced by C,
S 163 is replaced by C,
S 182 is replaced by C, and/or
N216 is replaced by C.

In one embodiment a cysteine is engineered into the fusion protein heavy chain variable domain, VH, for example:
S82b in SEQ ID NO:227 is replaced by C.

In one embodiment the antibody fusion protein heavy chain comprises an engineered cysteine wherein the position of said engineered cysteine is selected from the group consisting of S82b, T116, S163, S182 and N216.

All the residues specified herein above are derived from IgG1 but the disclosure herein also extends to corresponding Kabat positions in other heavy chains from other classes and isotypes of antibodies.

Accordingly, in one embodiment the antibody fusion protein heavy chain comprises an engineered cysteine wherein the position of said engineered cysteine is selected from the group consisting of position 3, 7, 8, 9, 10, 13, 15, 16, 17, 21, 26, 40, 43, 57, 58, 61, 62, 64, 65, 66, 68, 70, 72, 82a, 82b, 82c, 84, 85, 86, 96, 97, 98, 99, 105, 112, 113, 114, 115, 116, 117, 120, 127, 128, 129, 130, 133, 134, 135, 136, 156, 163, 164, 165, 167, 168, 169, 176, 177, 179, 180, 182, 183, 195, 196, 197, 199, 200, 203, 205, 213, 215, 216, 218, 220, 222 and 232 numbered according to the Kabat numbering system.

In one embodiment the antibody fusion protein heavy chain comprises an engineered cysteine wherein the position of said engineered cysteine is selected from the group consisting of position 3, 7, 8, 9, 10, 13, 15, 16, 17, 21, 26, 43, 57, 58, 61, 62, 64, 65, 66, 68, 70, 72, 82a, 82b, 85, 86, 96, 97, 98, 99, 105, 116, 117, 120, 127, 128, 129, 130, 133, 134, 135, 136, 156, 163, 164, 165, 167, 168, 169, 179, 182, 183, 195, 196, 197, 199, 200, 203, 205, 213, 215, 216, 218, 220, 222 and 232 numbered according to the Kabat numbering system.

In one embodiment the antibody fusion protein heavy chain comprises an engineered cysteine wherein the position of said engineered cysteine is selected from the group consisting of position 82b, 116, 163, 182 and 216 numbered according to the Kabat numbering system.

In one embodiment a recombinant fusion protein of the present invention is PEGylated through an engineered cysteine in the heavy chain at position 163 and/or 182.

In one embodiment the fusion protein comprises a free cysteine to which a PEG molecule is attached or to which a PEG molecule can be attached. In one embodiment the fusion protein or part thereof comprises the one or more sequences selected from:
LVTVCSASTKGPS (SEQ ID NO:198)
LVTVSCASTKGPS (SEQ ID NO:199)
LVTVSSCSTKGPS, (SEQ ID NO:200)and/or
HTFPCVLQSSGLYS. (SEQ ID NO:201)

In one embodiment provides a cysteine engineered fusion protein comprises a free cysteine and optionally comprises a sequence selected from one or more of the following:

| | |
|---|---|
| SLSASCGDRVT | (SEQ ID NO:202) |
| QKPGKCPKLLI | (SEQ ID NO:203) |
| EIKRTCAAPSV | (SEQ ID NO:204) |
| TCAAPCVFIFPP | (SEQ ID NO:205) |
| FIFPPCDEQLK | (SEQ ID NO:206) |
| DEQLKCGTASV | (SEQ ID NO:207) |
| FYPRECKVQWK | (SEQ ID NO:208) |
| WKVDNCLQSGN | (SEQ ID NO:209) |
| ALQSGCSQESV | (SEQ ID NO:210) |
| VTEQDCKDSTY | (SEQ ID NO:211) |
| | |
| GLSSPCTKSFN | (SEQ ID NO:212) |
| NWIRQCPGNK | (SEQ ID NO:213) |
| LNSCTTEDTAT | (SEQ ID NO:214) |
| GQGTLVTVSACSTKGPSVFPL | (SEQ ID NO:215) |
| HTFPCVLQSSGLYS | (SEQ ID NO:216) |
| | |
| HTFPACLQSSGLYS | (SEQ ID NO:217) |
| FLSVSCGGRVT | (SEQ ID NO:218) |
| QKPGNCPRLLI | (SEQ ID NO:219) |
| EIKRTCAAPSV | (SEQ ID NO:220) |
| FYPRECKVQWK | (SEQ ID NO:221) |
| | |
| VTEQDCKDSTY | (SEQ ID NO:222) |

In one embodiment there is provided a light chain comprising the sequence:

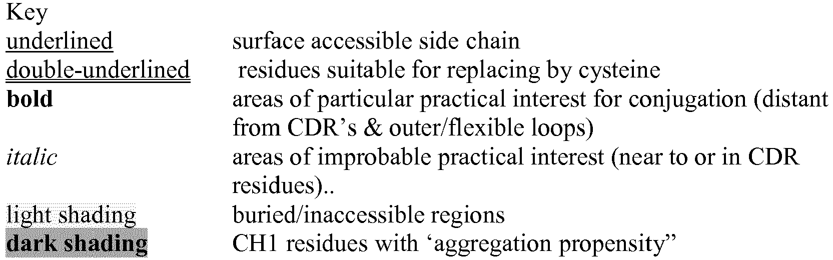

DIQMTQSPSSLSASVGDRVT²⁰ITCRASQDVNTAVAWYQQKP⁴⁰GKAPKLLIYSASFLE
SGVPS⁶⁰RFSGSRSGTDFTLTISSLQP⁸⁰EDFATYYCQQHYTTPPTFGQ¹⁰⁰GTKVEIKR
TVAAPSVFIFPP¹²⁰SDEQLKSGTASVVCLLNNFY¹⁴⁰PREAKVQWKVDNALQSGNSQ¹⁶
⁰ESVTEQDSKDSTYSLSSTLT¹⁸⁰LSKADYEKHKVYACEVTHQG²⁰⁰LSSPVTKSFNRGE
C²¹⁴

(SEQ ID NO:223)

In one embodiment there is provided a heavy chain comprising the sequence:

EVQLVESGGGLVQPGGSLRL²⁰SCAASGFNIKDTYIHWVRQA⁴⁰PGKGLEWVARIYPTN
GYTRY⁶⁰ADSVKGRFTISADTSKNTAY⁸⁰LQMNSLRAEDTAVYYCSRWG¹⁰⁰GDGFYAMD
YWGQGTLVTVSS¹²⁰ASTKGPSVFPLAPSSKSTSG¹⁴⁰GTAALGCLVKDYFPEPVTVS¹⁶
⁰WNSGALTSGVHTFPAVLQSS¹⁸⁰GLYSLSSVVTVPSSSLGTQT²⁰⁰YICNVNHKPSNTK
VDKKVEP²²⁰KSC
(SEQ ID NO:224)

In one embodiment the PEG polymer is conjugated to a position in a lower light chain variable domain, for example VL1 and/or VL2 suitably distant from CDR's, and near/adjacent to the elbow region but not encoded therewithin, such as Ser12, Ser14, Gln79, or Glu81, such that the binding of the of the entity is not in any way diminished or adversely affected. The numbering of these light chain residues is the same using Kabat numbering.

In one embodiment the PEG polymer is conjugated to a position in a lower heavy chain variable domain, for example VH1 and/or VH2, suitably distant from CDR's, and near/adjacent to the elbow region but not encoded therewithin, such as Gln13 (Gln 13 by Kabat numbering) or Glu89 (Glu85 by Kabat numbering), such that the binding of the the entity is not in any way diminished or adversely affected.

In one embodiment the heavy chain comprises the sequence given in SEQ ID NO:225 and/or 227.

In one embodiment the light chain comprises the sequence given in SEQ ID NO:228 and/or 44.

All embodiments herein are such that the conjugation of the PEG polymer to the entity does not diminish or adversely affect the binding/ affinity of the fusion protein of the disclosure.

Suitable polymers include a polyalkylene polymer, such as a poly(ethyleneglycol) or, especially, a methoxypoly(ethyleneglycol) or a derivative thereof, and especially with a molecular weight in the range from about 15000Da to about 40000Da. Other suitable polymers such as starches, complex glycoforms such as N-GlucNac and others eg Polymers investigated include those based on amino acids such as poly-GGGGS, polyglutamate and polyaspartate (Schlapschy et al., 2007; Jultani et al., 1997; Zunino et al., 1982); those based on carbohydrates such as oxidized dextran, carboxymethyl dextran, starch and polysialic acid (Fagnani et al., 1990; Baudys et al., 1998; Gregoriadis et al., 2000); and completely synthetic polymers such as poly(N-vinylpyrrolidone), poly(N-acryloilmorpholine), polyoxyethylated glycerol, hydroxypropyl methacrylamide, polymethacrylate, bow tie dendrimers and PEG (Kaneda et al., 2004; Caliceti et al., 1999; Soucek et al., 2002).

The size of the polymer may be varied as desired, but will generally be in an average molecular weight range from 500Da to 80000Da, for example from 5000 to 50000Da such as from 20000 to 40000Da. The polymer size may in particular be selected on the basis of the intended use of the product for example ability to localize to certain tissues such as tumors or extend circulating half-life (for review see Chapman, 2002, Advanced Drug Delivery Reviews, 54, 531-545). Thus, for example, where the product is intended to leave the circulation and penetrate tissue, for example for use in the treatment of a tumour, it may be advantageous to use a small molecular weight polymer, for example with a molecular weight of around 5000Da. For applications where the product remains in the circulation, it may be advantageous to use a higher molecular weight polymer, for example having a molecular weight in the range from 20000Da to 40000Da.

PEG molecules may be attached through any available amino acid side-chain or terminal amino acid functional group located in the antibody fragment, for example any free amino, imino, thiol, hydroxyl or carboxyl group. Such amino acids may occur naturally in the antibody fragment or may be engineered into the fragment using recombinant DNA methods (see for example US 5,219,996; US 5,667,425; WO98/25971). In one example the antibody molecule of the present invention is a modified Fab fragment wherein the modification is the addition to the C-terminal end of its heavy chain one or more amino acids to allow the attachment of an effector molecule. Suitably, the additional amino acids form a modified hinge region containing one or more cysteine residues to which the effector molecule may be attached. Multiple sites can be used to attach two or more PEG molecules.

The Fab or Fab' employed in the present invention is PEGylated with one or two PEG molecules.

In one embodiment a PEG molecule is linked to a cysteine 171 in the light chain, for example see WO2008/038024 incorporated herein by reference. The Fab or Fab' employed in the present invention is PEGylated through a solvent or surface accessible cysteine.

Suitably PEG molecules are covalently linked through a thiol group of at least one cysteine residue located in the fusion protein. Each polymer molecule attached to the fusion protein may be covalently linked to the sulphur atom of a cysteine residue located in the protein. The covalent linkage will generally be a disulphide bond or, in particular, a sulphur-carbon bond. Where a thiol group is used as the point of attachment appropriately activated PEG molecules, for example thiol selective derivatives such as maleimides and cysteine derivatives may be used. An activated PEG molecule may be used as the starting material in the preparation of polymer-fusion protein containing molecules as described above. The activated PEG molecule may be any polymer containing a thiol reactive group such as an α-halocarboxylic acid or ester, e.g. iodoacetamide, an imide, e.g. maleimide, a vinyl sulphone or a disulphide. Such starting materials may be obtained commercially (for example from Nektar, USA; Nippon Oils and Fats (NOF), Japan; Dr Reddy, UK; JenKem, China; Pan Asia Bio, China; SunBio, South Korea; Biovectra, USA) or may be prepared from commercially available starting materials using conventional chemical procedures. Particular PEG molecules include 20K methoxy-PEG-amine and 20K methoxy-PEG-N-hydroxy succinimde ester (for example from Nippon Oils and Fats (NOF), Japan; Dr Reddy, UK; JenKem, China; Pan Asia Bio, China; SunBio, South Korea; Rapp Polymere, Germany).

Effector molecules, such as PEG molecules, may be attached to fusion proteins by a number of different methods, including through aldehyde sugars or more commonly through any available amino acid side-chain or terminal amino acid functional group located in the antibody fragment, for example any free amino, imino, thiol, hydroxyl or carboxyl group. The site of attachment of effector molecules can be either random or site specific.

Random attachment is often achieved through amino acids such as lysine and this results in effector molecules, such as PEG molecules, being attached at a number of sites throughout the antibody fragment depending on the position of the lysines. While this has been successful in some cases the exact location and number of effector molecules, such as PEG molecules, attached cannot be controlled and this can lead to loss of activity for example if too few are attached and/or loss of affinity if for example they interfere with the antigen binding site (Chapman 2002 Advanced Drug Delivery Reviews, 54, 531-545). As a result, controlled site specific attachment of effector molecules, such as PEG molecules, is usually the method of choice.

Site specific attachment of effector molecules, such as PEG molecules, is most commonly achieved by attachment to cysteine residues since such residues are relatively uncommon in antibody fragments. Antibody hinges are popular regions for site specific attachment since these contain cysteine residues and are remote from other regions of the fusion protein likely to be involved in antigen binding. Suitable hinges either occur naturally in the fragment or may be created using recombinant DNA techniques (See for example US 5,677,425; WO98/25971; Leong et al., 2001 Cytokine, 16, 106-119; Chapman et al., 1999 Nature Biotechnology, 17, 780-783). Site-specific cysteines may also be engineered into the antibody fragment for example to create surface exposed cysteine(s) for effector molecule attachment (US 5,219,996).

In one embodiment the fusion protein according to the invention comprises a hinge as a linker between CL and VL2 spacer of approximately corresponding length to the hinge.

Techniques have been described in which native and engineered cysteines are used for the site-specific attachment of effector molecules, such as PEG molecules, (See WO2005003169, WO2005003170 and WO2005003171). In all of these fragments the native interchain disulphide bond between the heavy and light chain constant regions (CH1 and CL) is absent either because the interchain cysteines have been used as a site of attachment for effector molecules or because the interchain cysteines have been replaced by another amino acid to avoid effector molecule attachment to those cysteines. These fragments may also comprise engineered cysteines for use as sites of effector molecule attachment. In one example these engineered cysteines are a pair of engineered cysteines which form a disulphide link between the heavy and light chain constant regions of the antibody fragment starting material; said disulphide linkage is lost however once effector molecules are attached to those cysteines.

In one embodiment the fusion protein PEG molecule conjugates are provided in which the heavy and light chains of the antibody fragments are linked by an engineered interchain disulphide bond which is not the native interchain disulphide bond. This engineered interchain disulphide bond is retained during effector molecule attachment even when strong reducing agents are used. There are also provided sites in the light chain:heavy chain interface where pairs of cysteines can be successfully engineered to introduce a disulphide bond that is sufficiently buried that it is largely inaccessible to reducing agents and effector molecules, examples of 'buried disulphides' are shown in WO2007010231.

A particular advantage of these fragments lies in that the disulphide bond between the engineered interchain cysteines remains intact during effector molecule attachment.
A fusion protein may be madeto which one or more effector molecules is/are attached via the native interchain disulphide bond between the heavy (CH1) and light (CL) chain constant regions is absent and the heavy chain (CH1) and light chain (CL) constant regions are linked by an interchain disulphide bond between a pair of engineered cysteines, one in the light chain (CL) constant region and the other in the heavy chain constant (CH1) region.

In the present invention the PEG is conjugated to a surface accessible cysteine.

The term 'native interchain disulphide bond' as used herein refers to the interchain disulphide bond that exists between the cysteine in the heavy and light chain constant regions encoded in a naturally occurring germline antibody gene. In particular the native interchain cysteines are a cysteine in the constant region of the light chain (CL) and a cysteine in the first constant region of the heavy chain (CH1) that are disulphide linked to each other in naturally occurring antibodies. Examples of such cysteines may typically be found at position 214 of the light chain and 233 of the heavy chain of human IgG1, 127 of the heavy chain of human IgM, IgE, IgG2, IgG3, IgG4 and 128 of the heavy chain of human IgD and IgA2B, as defined by Kabat et al., 1987, in Sequences of Proteins of Immunological Interest, US Department of Health and Human Services, NIH, USA. It will be appreciated that the exact positions of these cysteines may vary from that of naturally occurring antibodies if any modifications, such as deletions, insertions and/or substitutions have been made to the antibody fragment.

Thus in one or more embodiment the native interchain disulphide bond is absent. The native interchain disulphide bond may be absent because one or more effector molecules are attached thereto.

In another embodiment the native interchain disulphide bond is absent in the antibody fragments of the present invention because the interchain cysteines have been replaced with another amino acid, such as serine.

In the antibody fragments of the present invention the heavy and light chain constant regions are linked by an interchain disulphide bond between an engineered cysteine in the light and/or heavy chain.

The fusion proteins of the present invention suitably have a high binding affinity, in particular picomolar. Affinity may be measured using any suitable method known in the art, including BIAcore. In one embodiment the antibody molecule of the present invention has a binding affinity of about 100 pM or better. In one embodiment the fusion protein of the present invention has a binding affinity of about 50pM or better. In one embodiment the fusion protein of the present invention has a binding affinity of about 40pM or better. In one embodiment the fusion protein of the present invention has a binding affinity of about 30pM or better. In one embodiment the fusion protein of the present invention is fully human or humanised and has a binding affinity of about 100pM or better.

If desired a fusion protein of the present invention may be conjugated to one or more further effector molecule(s). It will be appreciated that the effector molecule may comprise a single effector molecule or two or more such molecules so linked as to form a single moiety that can be attached to the antibodies of the present invention. Where it is desired to obtain an antibody fragment linked to an effector molecule, this may be prepared by standard chemical or recombinant DNA procedures in which the antibody fragment is linked either directly or via a coupling agent to the effector molecule. Techniques for conjugating such effector molecules to antibodies are well known in the art (see, Hellstrom et al., Controlled Drug Delivery, 2nd Ed., Robinson et al., eds., 1987, pp. 623-53; Thorpe et al., 1982 , Immunol. Rev., 62:119-58 and Dubowchik et al., 1999, Pharmacology and Therapeutics, 83, 67-123). Particular chemical procedures include, for example, those described in WO 93/06231, WO 92/22583, WO 89/00195, WO 89/01476 and WO03031581. Alternatively, where the effector molecule is a protein or polypeptide the linkage may be achieved using recombinant DNA procedures, for example as described in WO 86/01533 and EP0392745.

The term effector molecule as used herein includes, for example, antineoplastic agents, drugs, toxins, biologically active proteins, for example enzymes, other antibody or antibody fragments, synthetic or naturally occurring polymers, nucleic acids and fragments thereof e.g. DNA, RNA and fragments thereof, radionuclides, particularly radioiodide, radioisotopes, chelated metals, nanoparticles and reporter groups such as fluorescent compounds or compounds which may be detected by NMR or ESR spectroscopy.

Examples of effector molecules may include cytotoxins or cytotoxic agents including any agent that is detrimental to (*e.g.* kills) cells. Examples include combrestatins, dolastatins, epothilones, staurosporin, maytansinoids, spongistatins, rhizoxin, halichondrins, roridins, hemiasterlins, taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof.

Effector molecules also include, but are not limited to, antimetabolites (*e.g*. methotrexate, 6-mercaptopurine, 6-thioguanine, cytarabine, 5-fluorouracil decarbazine), alkylating agents (*e.g.* mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (*e.g.* daunorubicin (formerly daunomycin) and doxorubicin), antibiotics (*e.g.* dactinomycin (formerly actinomycin), bleomycin, mithramycin, anthramycin (AMC), calicheamicins or duocarmycins), and anti-mitotic agents (*e.g.* vincristine and vinblastine).

Other effector molecules may include chelated radionuclides such as ¹¹¹In and ⁹⁰Y, Lu¹⁷⁷, Bismuth²¹³, Californium²⁵², Iridium¹⁹² and Tungsten¹⁸⁸/Rhenium¹⁸⁸; or drugs such as but not limited to, alkylphosphocholines, topoisomerase I inhibitors, taxoids and suramin.

Other effector molecules include proteins, peptides and enzymes. Enzymes of interest include, but are not limited to, proteolytic enzymes, hydrolases, lyases, isomerases, transferases. Proteins, polypeptides and peptides of interest include, but are not limited to, immunoglobulins, toxins such as abrin, ricin A, pseudomonas exotoxin, or diphtheria toxin, a protein such as insulin, tumour necrosis factor, α-interferon, β-interferon, nerve growth factor, platelet derived growth factor or tissue plasminogen activator, a thrombotic agent or an anti-angiogenic agent, *e.g.* angiostatin or endostatin, or, a biological response modifier such as a lymphokine, interleukin-1 (IL-1), interleukin-2 (IL-2), granulocyte macrophage colony stimulating factor (GM-CSF), granulocyte colony stimulating factor (G-CSF), nerve growth factor (NGF) or other growth factor and immunoglobulins.

Other effector molecules may include detectable substances useful for example in diagnosis. Examples of detectable substances include various enzymes, prosthetic groups, fluorescent materials, luminescent materials, bioluminescent materials, radioactive nuclides, positron emitting metals (for use in positron emission tomography), and nonradioactive paramagnetic metal ions. See generally U.S. Patent No. 4,741,900 for metal ions which can be conjugated to antibodies for use as diagnostics. Suitable enzymes include horseradish peroxidase, alkaline phosphatase, beta-galactosidase, or acetylcholinesterase; suitable prosthetic groups include streptavidin, avidin and biotin; suitable fluorescent materials include umbelliferone, fluorescein, fluorescein isothiocyanate, rhodamine, dichlorotriazinylamine fluorescein, dansyl chloride and phycoerythrin; suitable luminescent materials include luminol; suitable bioluminescent materials include luciferase, luciferin, and aequorin; and suitable radioactive nuclides include ¹²⁵I, ¹³¹I, ¹¹¹In and ⁹⁹Tc.

In another example the effector molecule may increase the half-life of the antibody *in vivo,* and/or reduce immunogenicity of the antibody and/or enhance the delivery of an antibody across an epithelial barrier to the immune system. Examples of suitable effector molecules of this type include polymers, albumin, albumin binding proteins or albumin binding compounds such as those described in WO05/117984.

Where the effector molecule is a polymer it may, in general, be a synthetic or a naturally occurring polymer, for example an optionally substituted straight or branched chain polyalkylene, polyalkenylene or polyoxyalkylene polymer or a branched or unbranched polysaccharide, e.g. a homo- or hetero- polysaccharide.

Specific optional substituents which may be present on the above-mentioned synthetic polymers include one or more hydroxy, methyl or methoxy groups.

Specific examples of synthetic polymers include optionally substituted straight or branched chain poly(propyleneglycol) poly(vinylalcohol) or derivatives thereof, especially optionally substituted poly(ethyleneglycol) such as methoxypoly(ethyleneglycol) or derivatives thereof.
Specific naturally occurring polymers include lactose, amylose, dextran, glycogen or derivatives thereof.

"Derivatives" as used herein is intended to include reactive derivatives, for example thiol-selective reactive groups such as maleimides and the like. The reactive group may be linked directly or through a linker segment to the polymer. It will be appreciated that the residue of such a group will in some instances form part of the product as the linking group between the fusion protein and the polymer.

The present disclosure extends to isolated DNA encoding an antibody described herein or a fragment thereof of a heavy or light chain thereof.

The present disclosure includes a vector comprising said DNA.

General methods by which the vectors may be constructed, transfection methods and culture methods are well known to those skilled in the art. In this respect, reference is made to "Current Protocols in Molecular Biology", 1999, F. M. Ausubel (ed), Wiley Interscience, New York and the Maniatis Manual produced by Cold Spring Harbor Publishing.

The disclosure also includes a host cell comprising said vector and/or DNA.

Any suitable host cell/vector system may be used for expression of the DNA sequences encoding the fusion protein molecule of the present invention. Bacterial, for example *E. coli,* and other microbial systems may be used or eukaryotic, for example mammalian, host cell expression systems may also be used. Suitable mammalian host cells include CHO, myeloma or hybridoma cells. A process for the production of an fusion protein molecule according to the present invention comprises culturing a host cell containing a vector (and/or DNA) of the present invention under conditions suitable for leading to expression of protein from DNA encoding the antibody molecule of the present invention, and isolating the antibody molecule.

For production of products comprising both heavy and light chains, the cell line may be transfected with two vectors, a first vector encoding a light chain polypeptide and a second vector encoding a heavy chain polypeptide. Alternatively, a single vector may be used, the vector including sequences encoding light chain and heavy chain polypeptides.

The fusion protein molecules according to the present disclosure are expressed at good levels from host cells. Thus the properties of the fusion protein molecule are optimised and conducive to commercial processing.

The fusion protein molecules of the present invention are useful in the treatment and/or prophylaxis of a pathological condition.

Thus there is provided a fusion protein molecule for use in treatment, for example by administering a therapeutically effective amount thereof. In one embodiment the fusion protein molecules is administered in as a pharmaceutical formulation, comprising a pharmaceutically acceptable excipient.

Thus the present invention also provides a pharmaceutical or diagnostic composition comprising a fusion protein molecule of the present invention in combination with one or more of a pharmaceutically acceptable excipient, diluent or carrier. Accordingly, provided is the use of a fusion protein molecules of the invention for the manufacture of a medicament. The composition will usually be supplied as part of a sterile, pharmaceutical composition that will normally include a pharmaceutically acceptable carrier. A pharmaceutical composition of the present invention may additionally comprise a pharmaceutically-acceptable adjuvant.

The present disclosurealso provides a process for preparation of a pharmaceutical or diagnostic composition comprising adding and mixing the fusion protein molecule of the present invention together with one or more of a pharmaceutically acceptable excipient, diluent or carrier.

The fusion protein molecule may be the sole active ingredient in the pharmaceutical or diagnostic composition or may be accompanied by other active ingredients including other antibody ingredients, for example anti-TNF, anti- IL-1β, anti-T cell, anti-IFNγ or anti-LPS antibodies, or non-antibody ingredients such as xanthines. Other suitable active ingredients include antibodies capable of inducing tolerance, for example, anti-CD3 or anti-CD4 antibodies.

The fusion protein molecule or composition according to the disclosure is employed in combination with a further pharmaceutically active agent, for example a corticosteroid (such as fluticasonoe propionate) and/or a beta-2-agonist (such as salbutamol, salmeterol or formoterol) or inhibitors of cell growth and proliferation (such as rapamycin, cyclophosphmide, methotrexate) or alternative a CD28 and /or CD40 inhibitor. In one embodiment the inhitor is a small molecule. In another embodiment the inhibitor is an antibody specific to the target.

The pharmaceutical compositions suitably comprise a therapeutically effective amount of the antibody of the invention. The term "therapeutically effective amount" as used herein refers to an amount of a therapeutic agent needed to treat, ameliorate or prevent a targeted disease or condition, or to exhibit a detectable therapeutic or preventative effect. For any antibody, the therapeutically effective amount can be estimated initially either in cell culture assays or in animal models, usually in rodents, rabbits, dogs, pigs or primates. The animal model may also be used to determine the appropriate concentration range and route of administration. Such information can then be used to determine useful doses and routes for administration in humans.

The precise therapeutically effective amount for a human subject will depend upon the severity of the disease state, the general health of the subject, the age, weight and gender of the subject, diet, time and frequency of administration, drug combination(s), reaction sensitivities and tolerance/response to therapy. This amount can be determined by routine experimentation and is within the judgement of the clinician. Generally, a therapeutically effective amount will be from 0.01 mg/kg to 50 mg/kg, for example 0.1 mg/kg to 20 mg/kg. Pharmaceutical compositions may be conveniently presented in unit dose forms containing a predetermined amount of an active agent of the invention per dose.

Compositions may be administered individually to a patient or may be administered in combination (e.g. simultaneously, sequentially or separately) with other agents, drugs or hormones.

The dose at which the fusion protein molecule of the present invention is administered depends on the nature of the condition to be treated, the extent of the inflammation present and on whether the antibody molecule is being used prophylactically or to treat an existing condition.

The frequency of dose will depend on the half-life of the antibody molecule and the duration of its effect. If the antibody molecule has a short half-life (e.g. 2 to 10 hours) it may be necessary to give one or more doses per day. Alternatively, if the antibody molecule has a long half life (e.g. 2 to 15 days) it may only be necessary to give a dosage once per day, once per week or even once every 1 or 2 months.

The pharmaceutically acceptable carrier should not itself induce the production of antibodies harmful to the individual receiving the composition and should not be toxic. Suitable carriers may be large, slowly metabolised macromolecules such as proteins, polypeptides, liposomes, polysaccharides, polylactic acids, polyglycolic acids, polymeric amino acids, amino acid copolymers and inactive virus particles.

Pharmaceutically acceptable salts can be used, for example mineral acid salts, such as hydrochlorides, hydrobromides, phosphates and sulphates, or salts of organic acids, such as acetates, propionates, malonates and benzoates.

Pharmaceutically acceptable carriers in therapeutic compositions may additionally contain liquids such as water, saline, glycerol and ethanol. Additionally, auxiliary substances, such as wetting or emulsifying agents or pH buffering substances, may be present in such compositions. Such carriers enable the pharmaceutical compositions to be formulated as tablets, pills, dragees, capsules, liquids, gels, syrups, slurries and suspensions, for ingestion by the patient.

Suitable forms for administration include forms suitable for parenteral administration, e.g. by injection or infusion, for example by bolus injection or continuous infusion. Where the product is for injection or infusion, it may take the form of a suspension, solution or emulsion in an oily or aqueous vehicle and it may contain formulatory agents, such as suspending, preservative, stabilising and/or dispersing agents. Alternatively, the antibody molecule may be in dry form, for reconstitution before use with an appropriate sterile liquid.

Once formulated, the compositions can be administered directly to the subject. The subjects to be treated can be animals. However, in one or more embodiments the compositions are adapted for administration to human subjects.

Suitably in formulations according to the present disclosure, the pH of the final formulation is not similar to the value of the isoelectric point of the antibody or fragment, for example if the pH of the formulation is 7 then a pI of from 8-9 or above may be appropriate. Whilst not wishing to be bound by theory it is thought that this may ultimately provide a final formulation with improved stability, for example the antibody or fragment remains in solution.

The pharmaceutical compositions may be administered by any number of routes including, but not limited to, oral, intravenous, intramuscular, intra-arterial, intramedullary, intrathecal, intraventricular, transdermal, transcutaneous (for example, see WO98/20734), subcutaneous, intraperitoneal, intranasal, enteral, topical, sublingual, intravaginal or rectal routes. Hyposprays may also be used to administer the pharmaceutical compositions of the invention. Typically, the therapeutic compositions may be prepared as injectables, either as liquid solutions or suspensions. Solid forms suitable for solution in, or suspension in, liquid vehicles prior to injection may also be prepared.

Direct delivery of the compositions will generally be accomplished by injection, subcutaneously, intraperitoneally, intravenously or intramuscularly, or delivered to the interstitial space of a tissue. The compositions can also be administered into a lesion. Dosage treatment may be a single dose schedule or a multiple dose schedule.

It will be appreciated that the active ingredient in the composition will be a fusion protein molecule. As such, it will be susceptible to degradation in the gastrointestinal tract. Thus, if the composition is to be administered by a route using the gastrointestinal tract, the composition will need to contain agents which protect the antibody from degradation but which release the antibody once it has been absorbed from the gastrointestinal tract.

A thorough discussion of pharmaceutically acceptable carriers is available in Remington's Pharmaceutical Sciences (Mack Publishing Company, N.J. 1991). The formulation may be provided as a formulation for topical administrations including inhalation.

Suitable inhalable preparations include inhalable powders, metering aerosols containing propellant gases or inhalable solutions free from propellant gases. Inhalable powders according to the disclosure containing the active substance may consist solely of the abovementioned active substances or of a mixture of the abovementioned active substances with physiologically acceptable excipient.

These inhalable powders may include monosaccharides (e.g. glucose or arabinose), disaccharides (e.g. lactose, saccharose, maltose), oligo- and polysaccharides (e.g. dextranes), polyalcohols (e.g. sorbitol, mannitol, xylitol), salts (e.g. sodium chloride, calcium carbonate) or mixtures of these with one another. Mono- or disaccharides are suitably used, the use of lactose or glucose, particularly but not exclusively in the form of their hydrates.

Particles for deposition in the lung require a particle size less than 10 microns, such as 1-9 microns for example from 0.1 to 5 µm, in particular from 1 to 5 µm. The particle size of the active ingredient (such as the antibody or fragment) is of primary importance.

The propellent gases which can be used to prepare the inhalable aerosols are known in the art. Suitable propellent gases are selected from among hydrocarbons such as n-propane, n-butane or isobutane and halohydrocarbons such as chlorinated and/or fluorinated derivatives of methane, ethane, propane, butane, cyclopropane or cyclobutane. The abovementioned propellent gases may be used on their own or in mixtures thereof.

Particularly suitable propellent gases are halogenated alkane derivatives selected from among TG 11, TG 12, TG 134a and TG227. Of the abovementioned halogenated hydrocarbons, TG134a (1,1,1,2-tetrafluoroethane) and TG227 (1,1,1,2,3,3,3-heptafluoropropane) and mixtures thereof are particularly suitable.

The propellent-gas-containing inhalable aerosols may also contain other ingredients such as cosolvents, stabilisers, surface-active agents (surfactants), antioxidants, lubricants and means for adjusting the pH. All these ingredients are known in the art.

The propellant-gas-containing inhalable aerosols according to the invention may contain up to 5 % by weight of active substance. Aerosols according to the invention contain, for example, 0.002 to 5 % by weight, 0.01 to 3 % by weight, 0.015 to 2 % by weight, 0.1 to 2 % by weight, 0.5 to 2 % by weight or 0.5 to 1 % by weight of active ingredient.

Alternatively topical administrations to the lung may also be by administration of a liquid solution or suspension formulation, for example employing a device such as a nebulizer, for example, a nebulizer connected to a compressor (e.g., the Pari LC-Jet Plus(R) nebulizer connected to a Pari Master(R) compressor manufactured by Pari Respiratory Equipment, Inc., Richmond, Va.).

The fusion protein molecule of the invention can be delivered dispersed in a solvent, e.g., in the form of a solution or a suspension. It can be suspended in an appropriate physiological solution, e.g., saline or other pharmacologically acceptable solvent or a buffered solution. Buffered solutions known in the art may contain 0.05 mg to 0.15 mg disodium edetate, 8.0 mg to 9.0 mg NaCl, 0.15 mg to 0.25 mg polysorbate, 0.25 mg to 0.30 mg anhydrous citric acid, and 0.45 mg to 0.55 mg sodium citrate per 1 ml of water so as to achieve a pH of about 4.0 to 5.0. A suspension can employ, for example, lyophilised antibody.

The therapeutic suspensions or solution formulations can also contain one or more excipients. Excipients are well known in the art and include buffers (e.g., citrate buffer, phosphate buffer, acetate buffer and bicarbonate buffer), amino acids, urea, alcohols, ascorbic acid, phospholipids, proteins (e.g., serum albumin), EDTA, sodium chloride, liposomes, mannitol, sorbitol, and glycerol. Solutions or suspensions can be encapsulated in liposomes or biodegradable microspheres. The formulation will generally be provided in a substantially sterile form employing sterile manufacture processes.

This may include production and sterilization by filtration of the buffered solvent/solution used for the for the formulation, aseptic suspension of the antibody in the sterile buffered solvent solution, and dispensing of the formulation into sterile receptacles by methods familiar to those of ordinary skill in the art.

Nebulizable formulation according to the present disclosure may be provided, for example, as single dose units (e.g., sealed plastic containers or vials) packed in foil envelopes. Each vial contains a unit dose in a volume, e.g., 2 ml, of solvent/solution buffer.

The fusion protein molecule of the present disclosure are thought to be suitable for delivery via nebulisation.

Comprising in the context of the present specification is intended to meaning including.

Where technically appropriate embodiments of the invention may be combined.

Embodiments are described herein as comprising certain features/elements. The disclosure also extends to separate embodiments consisting or consisting essentially of said features/elements.

The present invention is further described by way of illustration only in the following examples, which refer to the accompanying Figures, in which:

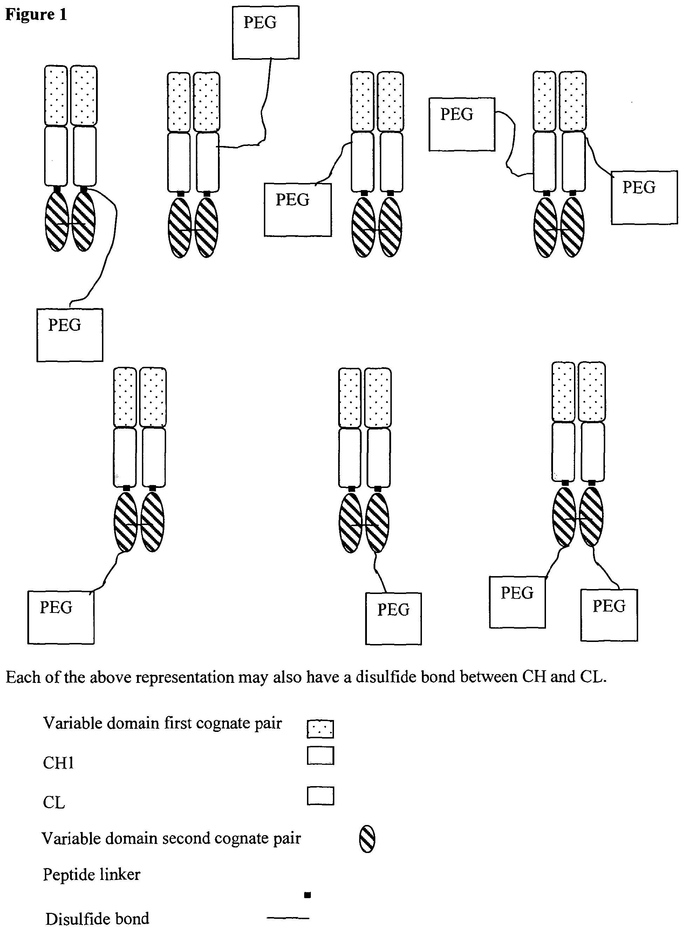

Figure 1 shows various fusion protein formats according to the present disclosure.
Figure 2A shows various known antibody fragments formats
Figure 2B shows one possible arrangement for a dimer format according to the presently claimed invention
Figure 3 shows the light chain amino acid sequence for antibody 4D5
Figure 4 shows the heavy chain amino acid sequence for antibody 4D5.

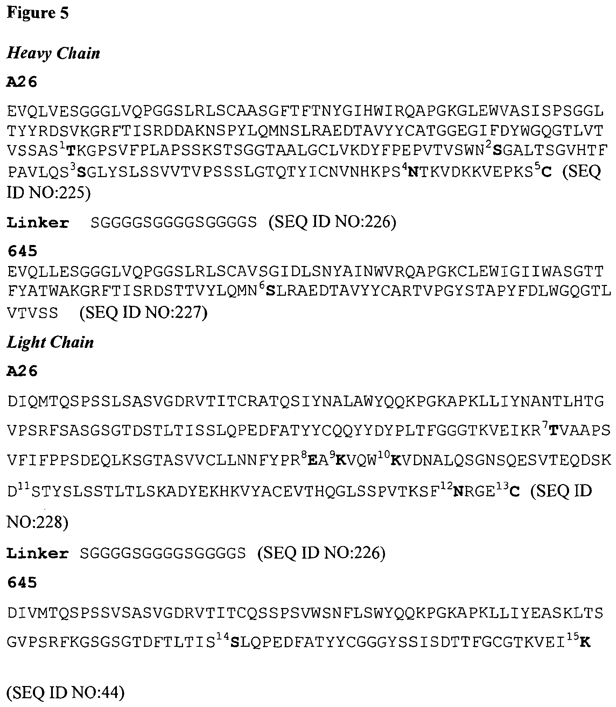

Figure 5 shows A26Fab-(3xG4S)-dsFv645 with positions of the surface Cys mutations shown in bold

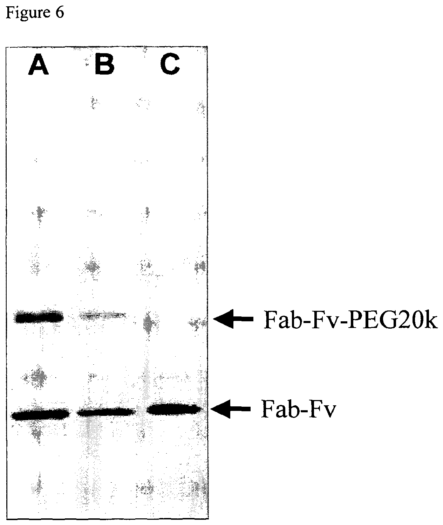

Figure 6 shows a non-reducing gel of PEGylated mutants of A26Fab-(3xG4S)-dsFv645, S182 and S163.

### Examples

### Fab-dsFv

Antibody fusions in the Fab-dsFv format were made as described in WO2010/035012. The Fab-dsFv comprised variable regions from the OX-40 antibody known as A26 described in WO2010/096418 and given in Figure 5 (SEQ ID NOS: 225 and 228) and the variable region sequences of anti-human serum albumin antibody known as 645 as described in WO2010/035012 and in Figure 5 SEQ ID NOs: 227 and 44. The format was A26Fab-ds645Fv.

Specific residues in the Fab-dsFv A26-645 were selected for the introduction of cysteine residues as PEGylation points. The selected mutations were in both the heavy and light chains and within both the Fab and the Fv. Care was taken to select residues away from the CDRs and any other structurally important motifs to limit the effect on protein production, folding, affinity and stability.

The selected residues are shown in Figure 5 and Table 4. The specific residues are numbered and highlighted in bold in Figure 5.

**Table 4.**

| Mutation number | Kabat numbering and domain |
|---|---|
| 1 | T116C CH1 |
| 2 | S163C CH1 |
| 3 | S182C CH1 |
| 4 | N216C CH1 |
| 5 | C233S CH1 |
| 6 | S82bC 645 Fv Heavy |
| 7 | T109C CK |
| 8 | E143C CK |
| 9 | K145C CK |
| 10 | K149C CK |
| 11 | S171C CK |
| 12 | N210C CK |
| 13 | C214S CK |
| 14 | S77C 645 Fv light |
| 15 | K107C 645 Fv light |

Mutations 5 and 13 were made as controls in which one of the two cysteine residues that contributes to the inter-chain disulphide bond was mutated to a serine while the other was left as a PEGylation site..

### Mutagenesis of Fab-dsFv

Site directed mutagenesis at each position in Table 4, numbered according to the Kabat numbering system, of the Fab-dsFv A26-645 was performed as follows: a vector encoding the Fab-dsFv A26-645 was subjected to site-directed mutagenesis using a standard 'overlapping PCR method'. Briefly, two mutagenic oligonucleotides were used along with two flanking oligonucleotides to generate two mutated PCR products in two separate PCR reactions. A small volume (typically ∼1ul) of crude overlapping PCR product from each reaction was used as a template in a 3^{rd} assembly PCR using the same two flanking oligonucleotides. This resulted in the generation of full length, mutated DNA sequence which was cloned by restriction / ligation into the expression plasmid before verification by DNA sequencing.

### Construction of Fab-dsFv fusion plasmids for expression in mammalian cells

Each Fab-dsFv heavy chain mutant was cloned into a mammalian expression vector under the control of the HCMV-MIE promoter and SV40E polyA sequence. These were paired with a similar vector containing the corresponding Fab-dsFv light chain for expression in mammalian cells (see below).

### Mammalian expression of Fab-dsFv

HEK293 cells were transfected with the heavy and light chain plasmids using Invitrogen's 293fectin transfection reagent according to the manufacturer's instructions. Briefly, 2µg heavy chain plasmid + 2µg light chain plasmid was incubated with 10µl 293fectin + 340µl Optimem media for 20mins at RT. The mixture was then added to 5x10⁶ HEK293 cells in suspension and incubated for 4 days with shaking at 37°C.

### Protein-G purification

The mammalian expression suspensions were clarified by centrifugation and the supernatants were concentrated to 2mL using 10kDa molecular weight cut off centrifugation concentrators. The concentrated supernatants were centrifuged at 16000xg for 10 min to remove any precipitate and then 1.8mL was loaded onto 1ml HiTrap Protein-G columns (GE Healthcare) at 1ml/min. The columns were washed with 20mM phosphate, 40mM NaCl pH7.4 and bound material eluted with 0.1M glycine/HCl pH2.7. The elution peak (2.5mL) was collected and pH adjusted to ∼pH7 with 100µL of 2M Tris/HCl pH8.5. The pH adjusted elutions were diafiltered into PBS using 10kDa molecular weight cut off centrifugation concentrators and concentrated to ∼500µL.

All the Fab-dsFvs, except for Thr109Cys and Asn216Cys, expressed reasonably well. The DNA sequences of both poor expressing mutants were verified, as there was no problem with the sequences yet both had very low yield, neither Thr109Cys on the light chain nor Asn211Cys on the heavy chain were analysed further.

### Biacore

Binding affinities and kinetic parameters for the interactions of Fab-dsFv constructs were determined by surface plasmon resonance (SPR) conducted on a Biacore 3000 using CM5 sensor chips and HBS-EP (10mM HEPES (pH7.4), 150mM NaCl, 3mM EDTA, 0.05% v/v surfactant P20) running buffer. Fab-dsFv samples were captured to the sensor chip surface using either a human F(ab')₂-specific goat Fab (Jackson ImmunoResearch, 109-006-097) or an in-house generated anti human CH1 monoclonal antibody. Covalent immobilisation of the capture antibody was achieved by standard amine coupling chemistry.

Each assay cycle consisted of firstly capturing the Fab-dsFv using a 1 min injection, before an association phase consisting of a 3 min injection of antigen, after which dissociation was monitored for 20 min. After each cycle, the capture surface was regenerated with 2 x 1 min injections of 40mM HCl followed by 30s of 5mM NaOH. The flow rates used were 10µl/min for capture, 30µl/min for association and dissociation phases, and 10µl/min for regeneration.

For kinetic assays, a titration of antigen was performed, a blank flow-cell was used for reference subtraction and buffer-blank injections were included to subtract instrument noise and drift.

Kinetic parameters were determined by simultaneous global-fitting of the resulting sensorgrams to a standard 1:1 binding model using Biacore 3000 Evaluation software.

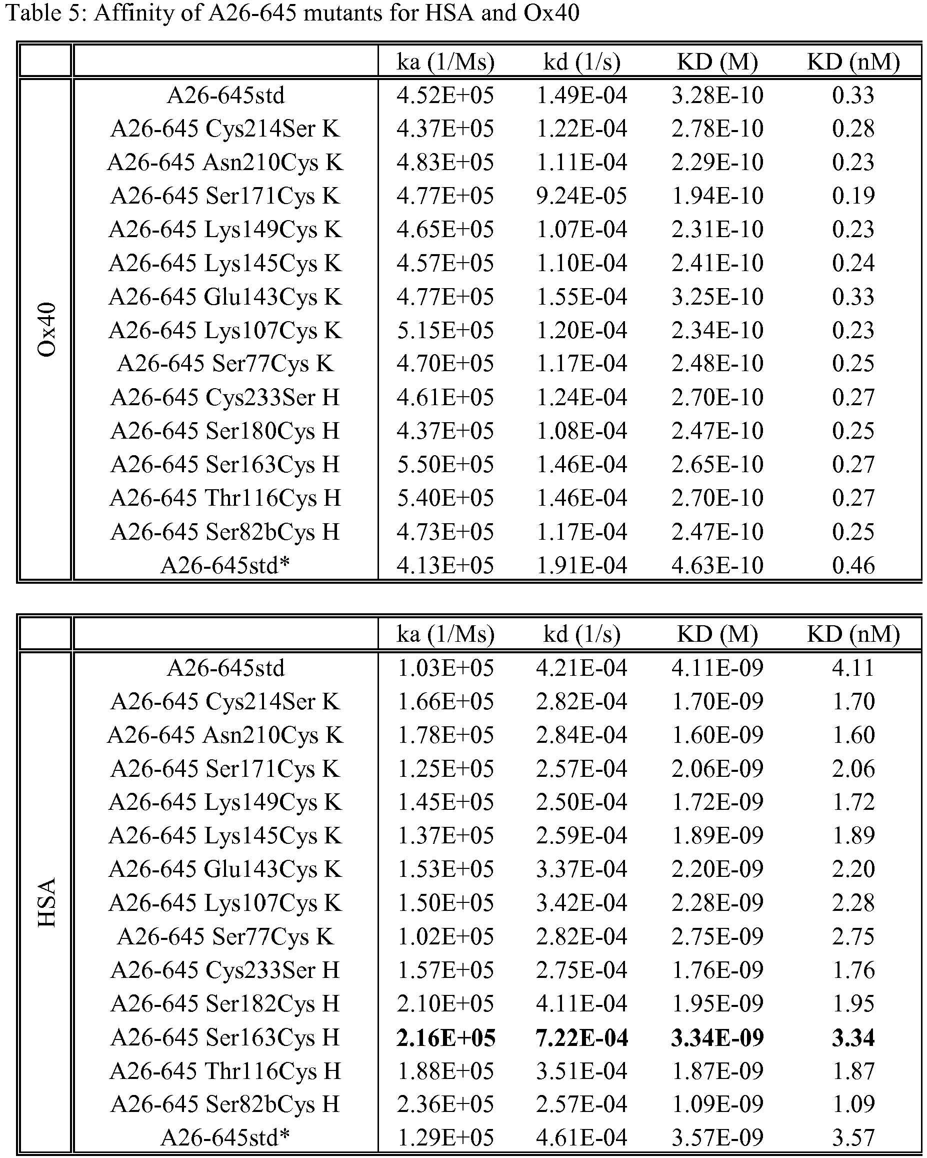

### Reduction and PEGylation of Fab-dsFv-Cys

Protein-G purified Fab-dsFv containing introduced cysteine PEGylation sites at S182C or S163C on CH1 in 0.1M phosphate, 2mM EDTA pH6 were reduced by the addition 100mM β-mercaptoethylamine (βMEA) in 0.1M phosphate, 2mM EDTA pH6 to a final concentration of 1mM. The reduction reactions were incubated for 1 hour at ambient temperature before being fractionated by SE-HPLC on a Sephacryl-200 10/30 column run in an isocratic gradient of 0.1M phosphate, 2mM EDTA pH6. The 500µl fraction containing the most monomer Fab-dsFv was identified and split into two. To one half was added 5µl of 150mg/ml SUNBRIGHT ME-200MA PEG (NOF) in 0.1M phosphate, 2mM EDTA pH6, this is a 20kDa, maleimide active PEG. To the other half was added 5µl of 100mM N-ethylmaleimide (NEM) in 0.1M phosphate, 2mM EDTA pH6. All samples were incubated overnight at ambient temperature. The samples were analysed on a non-reducing 4-20% acrylimide Tris/glycine gel. The gel was silver stained using an Owl Silver Stain kit as described in the manufactures instructions. See figure 6.
Figure 6
A = S182C CH1 + PEG
B = S163C CH1 + PEG
C = S182C CH1 + NEM

Lane C of figure 6 demonstrates that that the reduction does not break the intra and inter disulphide bonds within the Fab-dsFv. The only band present runs at the same position as a correctly disulphide bonded Fab-dsFv when compared to a standard that has not undergone reduction and NEM capping (data not shown). In lanes A and B there are 2 bands, the lower one corresponds to the correctly disulphide bonded Fab-dsFv and the higher one is a PEGylated correctly disulphide bonded Fab-dsFv. The shift on the gel is indicative of the addition of one 20k PEG. Due to the maleimide chemistry used, this data demonstrates site specific PEGylation of Fab-dsFv at the introduced cysteine PEGylation sites of S182C and S163C on CH1.

## Claims

1. A recombinant fusion protein comprising:
a heavy chain consisting of, in sequence from the N-terminal, a variable domain nominally V_{H}1, a C_{H}1 region and a further variable domain nominally V_{H}2,
a light chain consisting of, in sequence from the N-terminal, a variable domain
nominally V_{L}1, a CL domain and a variable domain nominally V_{L}2,
wherein said heavy and light chains are aligned to provide a first binding site formed by a first variable domain pair of V_{H}1 and V_{L}1 and a second binding site formed by a second variable domain pair of V_{H}2 and V_{L}2,
wherein there is a disulfide bond between the second variable domain pair of V_{H}2 and V_{L}2 between two cysteine residues selected from the group consisting of VH37 and VL95, VH44 and VL100, VH44 and VL105, VH45 and VL87, VH55 and VL101, VH100 and VL50, VH100b and VL49, VH98 and VL46, and, VH105 and VL43, such as wherein the cysteine of V_{H}2 is at position 44 and the cysteine of V_{L}2 is at position 100, and
said fusion protein is conjugated to one or two PEG polymer molecules through a solvent exposed cysteine, wherein the numbering is Kabat numbering of variable domains.

2. A recombinant fusion protein according to claim 1, wherein there is a disulphide bond between the first variable domain pair of V_{H}1 and V_{L}1.

3. A recombinant fusion protein according to any one of claims 1 to 2, wherein an amino acid of V_{H}2 is directly linked to an amino acid of C_{H}1 by a peptide bond.

4. A recombinant fusion protein according to claim 1, wherein V_{H}2 is linked to C_{H}1 indirectly by a linker.

5. A recombinant fusion protein according to any one of claims 1 to 4, wherein V_{L}2 is directly linked to an amino acid of CL by a peptide bond.

6. A recombinant fusion protein according to any one of claims 1 to 4 wherein V_{L}2 is linked to CL indirectly by a linker.

7. A recombinant fusion protein according to any one of claims 1 to 6, wherein the recombinant fusion protein is PEGylated through an engineered cysteine in the light chain wherein the position of said engineered cysteine is selected from the group consisting of position 5, 7, 9, 10, 12, 14, 15, 16, 17, 18, 20, 22, 24, 26, 27, 28, 30, 31,34, 39, 41, 42, 43, 55, 56, 57, 60, 61, 63, 67, 68, 69, 70, 72, 74, 76, 77, 79, 81, 107, 108, 109, 110, 114, 121, 122, 123, 126, 127, 128, 129, 143, 144, 145, 147, 149, 151, 152, 153, 156, 157, 158, 159, 160, 161, 167, 168, 169, 170, 171, 190, 195, 197, 199, 200, 202, 203, 205, 206, 210, 211, 212 and 213, numbered according to the Kabat numbering system, such as wherein the recombinant fusion protein is PEGylated through an engineered cysteine in the light chain wherein the position of said engineered cysteine is selected from the group consisting of position 77, 107, 109, 143, 145, 149 and 210, numbered according to the Kabat numbering system.

8. A recombinant fusion protein according to any one of claims 1 to 7, wherein the recombinant fusion protein is PEGylated through an engineered cysteine in the heavy chain wherein the position of said engineered cysteine is selected from the group consisting of position 3, 7, 8, 9, 10, 13, 15, 16, 17, 21, 26, 40, 43, 57, 58, 61, 62, 64, 65, 66, 68, 70, 72, 82a, 82b, 82c, 84, 85, 86, 96, 97, 98, 99, 105, 112, 113, 114, 115, 116, 117, 120, 127, 128, 129, 130, 133, 134, 135, 136, 156, 163, 164, 165, 167, 168, 169, 176, 177, 179, 180, 182, 183, 195, 196, 197, 199, 200, 203, 205, 213, 215, 216, 218, 220, 222 and 232, numbered according to the Kabat numbering system, such as wherein the recombinant fusion protein is PEGylated through an engineered cysteine in the heavy chain wherein the position of said engineered cysteine is selected from the group consisting of position 82b, 116, 163, 182 and 216, numbered according to the Kabat numbering system.

9. A recombinant fusion protein according to any one of claims 1 to 8, wherein the PEG molecules are in the range 5000 to 80000Da.

10. A recombinant fusion protein according to any one of claims 1 to 9, wherein the two variable domain pairs are each a cognate pair.

11. A recombinant fusion protein according to any one of claims 1 to 10, which is dimerised, wherein dimerization is selected from
a) dimerised directly via a disulphide bond, and
b) dimerised via a chemical linker, for example dimerised via a PEG linker or a dimerised via a peptide linker.

12. A recombinant fusion protein according to claim 11, which is dimerised via solvent accessible cysteines.

13. A pharmaceutical or diagnostic composition comprising a recombinant fusion protein according to any one of claims 1 to 12 and a pharmaceutically acceptable excipient, diluent or carrier.

14. A recombinant fusion protein according to any one of claims 1 to 12 or a composition according to claim 13, for use as a medicament.

## Patentansprüche

1. Rekombinantes Fusionsprotein, umfassend:
eine schwere Kette, bestehend aus, der Reihe nach vom N-Terminus, einer variablen Domäne, nominell V_{H}1, einer C_{H}1-Region und einer weiteren variablen Domäne, nominell V_{H}2, eine leichten Kette, bestehend aus, der Reihe nach vom N-Terminus, einer variablen Domäne, nominell V_{L}1, einer CL-Domäne und einer variablen Domäne, nominell V_{L}2,
wobei die schweren und leichten Ketten ausgerichtet sind, um eine erste Bindungsstelle, welche durch ein erstes Paar von V_{H}1 und V_{L}1 der variablen Domäne gebildet wird, und eine zweite Bindungsstelle, welche durch ein zweites Paar von V_{H}2 und V_{L}2 der variablen Domäne gebildet wird, bereitzustellen;
wobei es eine Disulfidbrücke zwischen dem zweiten Paar der variablen Domäne von V_{H}2 und V_{L}2 zwischen zwei Cysteinresten gibt, ausgewählt aus der Gruppe bestehend aus VH37 und VL95, VH44 und VL100, VH44 und VL105, VH45 und VL87, VH55 und VL101, VH100 und VL50, VH100b und VL49, VH98 und VL46 und VH105 und VL43, wobei beispielsweise das Cystein von V_{H}2 an Position 44 ist und das Cystein von V_{L}2 an Position 100 ist, und
das Fusionsprotein an ein oder zwei PEG-Polymermoleküle über ein Solvens-ausgesetztes Cystein konjugiert ist, wobei die Nummerierung der variablen Domänen die Kabat-Nummerierung ist.

2. Rekombinantes Fusionsprotein gemäß Anspruch 1, wobei es eine Disulfidbrücke zwischen dem ersten Paar von V_{H}1 und V_{L}1 der variablen Domäne gibt.

3. Rekombinantes Fusionsprotein gemäß einem der Ansprüche 1 bis 2, wobei eine Aminosäure von V_{H}2 direkt mit einer Aminosäure von C_{H}1 durch eine Peptidbindung verknüpft ist.

4. Rekombinantes Fusionsprotein gemäß Anspruch 1, wobei V_{H}2 indirekt über einen Linker mit C_{H}1 verknüpft ist.

5. Rekombinantes Fusionsprotein gemäß einem der Ansprüche 1 bis 4, wobei V_{L}2 direkt über eine Peptidbindung mit einer Aminosäure von CL verknüpft ist.

6. Rekombinantes Fusionsprotein gemäß einem der Ansprüche 1 bis 4, wobei V_{L}2 indirekt über einen Linker mit CL verknüpft ist.

7. Rekombinantes Fusionsprotein gemäß einem der Ansprüche 1 bis 6, wobei das rekombinante Fusionsprotein durch ein konstruiertes Cystein in der leichten Kette PEGyliert ist, wobei die Position des konstruierten Cysteins ausgewählt ist aus der Gruppe, bestehend aus Position 5, 7, 9, 10, 12, 14, 15, 16, 17, 18, 20, 22, 24, 26, 27, 28, 30, 31, 34, 39, 41, 42, 43, 55, 56, 57, 60, 61, 63, 67, 68, 69, 70, 72, 74, 76, 77, 79, 81, 107, 108, 109, 110, 114, 121, 122 123, 126, 127, 128, 129, 143, 144, 145, 147, 149, 151, 152, 153, 156, 157, 158, 159, 160, 161, 167, 168, 169, 170, 171, 190, 195, 197, 199, 200, 202, 203, 205, 206, 210, 211, 212 und 213, nummeriert gemäß dem Kabat-Nummerierungssystem, wobei beispielsweise das rekombinante Fusionsprotein durch ein konstruiertes Cystein in der leichten Kette PEGyliert ist, wobei die Position des konstruierten Cysteins ausgewählt ist aus der Gruppe, bestehend aus Position 77, 107, 109, 143, 145, 149 und 210, nummeriert gemäß dem Kabat-Nummerierungssystem.

8. Rekombinantes Fusionsprotein gemäß einem der Ansprüche 1 bis 7, wobei das rekombinante Fusionsprotein durch ein konstruiertes Cystein in der schweren Kette PEGyliert ist, wobei die Position des konstruierten Cysteins ausgewählt ist aus der Gruppe, bestehend aus Position 3, 7, 8, 9, 10, 13, 15, 16, 17, 21, 26, 40, 43, 57, 58, 61, 62, 64, 65, 66, 68, 70, 72, 82a, 82b, 82c, 84, 85, 86, 96, 97, 98, 99, 105, 112, 113, 114, 115, 116, 117, 120, 127, 128, 129, 130, 133, 134, 135, 136, 156, 163, 164, 165, 167, 168, 169, 176, 177, 179, 180, 182, 183, 195, 196, 197, 199, 200, 203, 205, 213, 215, 216, 218, 220, 222 und 232, nummeriert gemäß dem Kabat-Nummerierungssystem, wobei beispielsweise das rekombinante Fusionsprotein durch ein konstruiertes Cystein in der schweren Kette PEGyliert ist, wobei die Position des konstruierten Cysteins ausgewählt ist aus der Gruppe, bestehend aus Position 82b, 116, 163, 182 und 216, nummeriert gemäß dem Kabat-Nummerierungssystem.

9. Rekombinantes Fusionsprotein gemäß einem der Ansprüche 1 bis 8, wobei die PEG-Moleküle im Bereich von 5.000 bis 80.000 Da liegen.

10. Rekombinantes Fusionsprotein gemäß einem der Ansprüche 1 bis 9, wobei die zwei Paare der variablen Domäne jeweils ein verwandtes Paar sind.

11. Rekombinantes Fusionsprotein gemäß einem der Ansprüche 1 bis 10, das dimerisiert ist, wobei die Dimerisierung ausgewählt ist aus
a) direkt dimerisiert über eine Disulfidbrücke, und
b) über einen chemischen Linker dimerisiert, zum Beispiel dimerisiert über einen PEG-Linker oder dimerisiert über einen Peptid-Linker.

12. Rekombinantes Fusionsprotein gemäß Anspruch 11, das über Solvens-zugängliche Cysteine dimerisiert ist.

13. Pharmazeutische oder diagnostische Zusammensetzung, welche ein rekombinantes Fusionsprotein gemäß einem der Ansprüche 1 bis 12 und einen pharmazeutisch verträglichen Hilfsstoff, ein pharmazeutisch verträgliches Verdünnungsmittel oder einen pharmazeutisch verträglichen Träger umfasst.

14. Rekombinantes Fusionsprotein gemäß einem der Ansprüche 1 bis 12 oder eine Zusammensetzung gemäß Anspruch 13, zur Verwendung als Arzneimittel.

## Revendications

1. Protéine de fusion recombinée comprenant :
une chaîne lourde constituée, successivement à partir de la terminaison N, d'un domaine variable nominalement V_{H}1, d'une région C_{H}1 et d'un autre domaine variable, nominalement V_{H}2, une chaîne légère constituée, successivement à partir de la terminaison N, d'un domaine variable nominalement V_{L}1, d'un domaine CL et d'un domaine variable, nominalement V_{L}2,
dans laquelle lesdites chaînes lourde et légère sont alignées pour fournir un premier site de liaison formé par une première paire de domaines variables de V_{H}1 et V_{L}1 et un second site de liaison formé par une seconde paire de domaines variables de V_{H}2 et V_{L}2,
dans laquelle il y a une liaison disulfure entre la seconde paire de domaines variables de V_{H}2 et V_{L}2 entre deux résidus de cystéine choisis dans le groupe constitué de VH37 et VL95, VH44 et VL100, VH44 et VL105, VH45 et VL87, VH55 et VL101, VH100 et VL50, VH100b et VL49, VH98 et VL46, et VH105 et VL43, par exemple, dans laquelle la cystéine de V_{H}2 est à la position 44 et la cystéine de V_{L}2 est à la position 100, et
ladite protéine de fusion est conjuguée à une ou deux molécules de polymère de PEG par le biais d'une cystéine exposée à un solvant, dans laquelle la numérotation est la numérotation de Kabat des domaines variables.

2. Protéine de fusion recombinée selon la revendication 1, dans laquelle il y a une liaison disulfure entre la première paire de domaines variables de V_{H}1 et V_{L}1.

3. Protéine de fusion recombinée selon l'une quelconque des revendications 1 à 2, dans laquelle un acide aminé de V_{H}2 est directement lié à un acide aminé de C_{H}1 par une liaison peptidique.

4. Protéine de fusion recombinée selon la revendication 1, dans laquelle V_{H}2 est lié à C_{H}1 indirectement par une séquence de liaison.

5. Protéine de fusion recombinée selon l'une quelconque des revendications 1 à 4, dans laquelle V_{L}2 est directement lié à un acide aminé de CL par une liaison peptidique.

6. Protéine de fusion recombinée selon l'une quelconque des revendications 1 à 4, dans laquelle V_{L}2 est lié à CL indirectement par une séquence de liaison.

7. Protéine de fusion recombinée selon l'une quelconque des revendications 1 à 6, où la protéine de fusion recombinée est pégylée par le biais d'une cystéine artificiellement modifiée dans la chaîne légère, dans laquelle la position de ladite cystéine artificiellement modifiée est choisie dans le groupe constitué des positions 5, 7, 9, 10, 12, 14, 15, 16, 17, 18, 20, 22, 24, 26, 27, 28, 30, 31,34, 39, 41, 42, 43, 55, 56, 57, 60, 61, 63, 67, 68, 69, 70, 72, 74, 76, 77, 79, 81, 107, 108, 109, 110, 114, 121, 122, 123, 126, 127, 128, 129, 143, 144, 145, 147, 149, 151, 152, 153, 156, 157, 158, 159, 160, 161, 167, 168, 169, 170, 171, 190, 195, 197, 199, 200, 202, 203, 205, 206, 210, 211, 212 et 213, numérotées selon le système de numérotation de Kabat, par exemple, où la protéine de fusion recombinée est pégylée par le biais d'une cystéine artificiellement modifiée dans la chaîne légère dans laquelle la position de ladite cystéine artificiellement modifiée est choisie dans le groupe constitué des positions 77, 107, 109, 143, 145, 149 et 210, numérotées selon le système de numérotation de Kabat.

8. Protéine de fusion recombinée selon l'une quelconque des revendications 1 à 7, où la protéine de fusion recombinée es pégylée par le biais d'une cystéine artificiellement modifiée dans la chaîne lourde dans laquelle la position de ladite cystéine artificiellement modifiée est choisie dans le groupe constitué des positions 3, 7, 8, 9, 10, 13, 15, 16, 17, 21, 26, 40, 43, 57, 58, 61, 62, 64, 65, 66, 68, 70, 72, 82a, 82b, 82c, 84, 85, 86, 96, 97, 98, 99, 105, 112, 113, 114, 115, 116, 117, 120, 127, 128, 129, 130, 133, 134, 135, 136, 156, 163, 164, 165, 167, 168, 169, 176, 177, 179, 180, 182, 183, 195, 196, 197, 199, 200, 203, 205, 213, 215, 216, 218, 220, 222 et 232, numérotées selon le système de numérotation de Kabat, par exemple, où la protéine de fusion recombinée est pégylée par le biais d'une cystéine artificiellement modifiée dans la chaîne lourde dans laquelle la position de ladite cystéine artificiellement modifiée est choisie dans le groupe constitué des positions 82b, 116, 163, 182 et 216, numérotées selon le système de numérotation de Kabat.

9. Protéine de fusion recombinée selon l'une quelconque des revendications 1 à 8, dans laquelle les molécules de PEG sont dans la plage de 5000 à 80 000 Da.

10. Protéine de fusion recombinée selon l'une quelconque des revendications 1 à 9, dans laquelle les deux paires de domaines variables sont chacune une paire apparentée.

11. Protéine de fusion recombinée selon l'une quelconque des revendications 1 à 10, qui est dimérisée, dans laquelle la dimérisation est choisie parmi
a) dimérisée directement par l'intermédiaire d'une liaison disulfure, et
b) dimérisée par l'intermédiaire d'une séquence de liaison chimique, par exemple, dimérisée par l'intermédiaire d'une séquence de liaison PEG ou dimérisée par l'intermédiaire d'une séquence de liaison peptidique.

12. Protéine de fusion recombinée selon la revendication 11, qui est dimérisée par l'intermédiaire de cystéines accessibles à un solvant.

13. Composition pharmaceutique ou de diagnostic comprenant une protéine de fusion recombinée selon l'une quelconque des revendications 1 à 12 et un excipient, diluant ou support acceptable sur le plan pharmaceutique.

14. Protéine de fusion recombinée selon l'une quelconque des revendications 1 à 12 ou composition selon la revendication 13, pour une utilisation en tant que médicament.
